# EUROPEAN PATENT APPLICATION

(11) **EP 4 617 267 A1**
(43) Date of publication of application: **17.09.2025**
(21) Application number: 23888088.4
(22) Date of filing: 09.11.2023
(51) Int. Cl.: C07D 401/12, C07D 417/04, C07D 417/12, A61K 31/40, A61K 31/4439, A61P 29/00, A61P 35/00, A61P 17/04

(54) **THIOETHER COMPOUND HAVING SOFT DRUG PROPERTY AND USE THEREOF, AND PHARMACEUTICAL COMPOSITION AND USE THEREOF**

(30) Priority: 10.11.2022 CN 202211408345
(71) Applicant: Prime Gene Therapeutics Co., Ltd., Beijing 100070 (CN)
(72) Inventor: YANG, Yanqing, Beijing 100070 (CN); ZHU, Li, Beijing 100070 (CN); LI, Yuliang, Beijing 100070 (CN); ZHANG, Hui, Beijing 100070 (CN); DU, Daniel Yunlong, Beijing 100070 (CN)
(74) Representative: Winter, Brandl - Partnerschaft mbB
(86) International application number: PCT/CN2023/130830
(87) International publication number: WO 2024/099403

(57) **Abstract**

The present invention relates to a thioether compound having soft drug properties, and a pharmaceutical composition and use thereof. The compound has a structure of formula I. The thioether structure of the compound of the present invention has pharmacological activity, and after exerting pharmacological effects, it is rapidly converted into sulfoxide and sulfone metabolites that do not have pharmacological activity according to an established metabolic pathway, so that it can be achieved that the toxicity of prototype drugs, active metabolites and reactive metabolites to a body is avoided while exerting therapeutic effects, and the safety is relatively higher

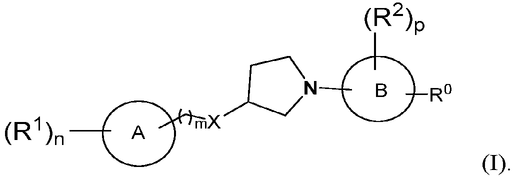

## Description

### Technical Field

The present invention relates to pharmaceutical field, and specifically, to a TRPV3 antagonist compound; a stereoisomer, and a pharmaceutical composition comprising the compound; as well as use of the compound and the pharmaceutical composition.

### Background

Transient receptor potential (TRP) is a type of ion channel protein that exists on cell membrane or intracellular organelle membrane, which consists of seven subfamilies: TRPC, TRPV, TRPM, TRPML, TRPP, TRPA, TRPN, etc. Moreover, the transient receptor potential vanilloid (TRPV) subfamily of mammals is in turns composed of TRPV1-6. In recent years, research has found that TRPV3 is mainly expressed in the keratinocytes of human skin, plays an important role in mediating skin sensation, influencing the proliferation and differentiation of epidermal keratinocytes, hair growth, participating in inflammatory responses, and maintaining skin homeostasis and normal function. This ion channel can be regulated by many factors, such as temperature, osmotic pressure, pH value, mechanical force, and intracellular signaling molecules. Research has shown that TRPV3 is a pathogenic gene for Olmsted syndrome, a rare skin disease (Am. J. Hum. Genet. 2012, 90, 558), and TRPV3 inhibitors have potential therapeutic prospects in keratinizing skin diseases, pruritic skin diseases, inflammation, abnormal hair growth, and painful skin diseases. TRPV3 is mainly expressed in skin keratinocytes, as well as in tissues such as tongue, dorsal root ganglion, trigeminal ganglion, spinal cord, and brain, and mainly responds to warm stimulation (32~39°C). The thermal sensitivity of TRPV3 is also regulated by calcium in extracellular fluid. When subjected to repeated thermal stimulation, the channel current continuously increases. Free nerve endings under the skin may sense and transmit thermal stimulation through signaling molecules similar to those present in thermoreceptive neurons. Therefore, TRPV3 has potential therapeutic prospects in painful diseases.

TRPV3 can be activated by monoterpenoid compounds (e.g., camphor, borneol, peppermint, etc.) and research has found that it works by increasing the level of intracellular divalent calcium ions (Ca²⁺). These aromatic compounds have anti-inflammatory, analgesic, and anti itch effects etc., and have been widely used in fields such as medicine and cosmetics. However, these early TRPV3 inhibitors were either mostly natural products with poor specificity and high effective doses, which could lead to serious side effects, or had mediocre molecular backbone activity, and research and development were mostly stalled. The existing TRPV3 inhibitors have the problem of limited structural types and slow development. It is necessary to develop molecules with novel scaffolds and clinical use value.

### Summary of the Invention

The present invention relates to a compound of formula (I) or stereoisomers, tautomers, solvates, hydrates, active metabolites, isotopic labels, and pharmaceutically acceptable salts thereof: 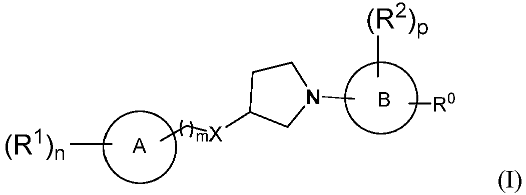
wherein ring A and ring B are each independently selected from a monocyclic or polycyclic ring system containing 3-12 ring atoms;
R¹ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}), -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R², together with the atoms of ring B to which they are connected, form a 3-10 membered ring structure;
R⁰ is each independently selected from the group consisting of H, halogen or a structure of formula II 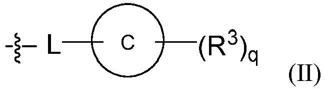
wherein,
L is each independently selected from the group consisting of a bond, -O-, -S-, -N(R²⁰)-, - C(O)-, -C(R²⁰R²¹)-, -S(O)- and -S(O₂)-;
ring C is each independently selected from a monocyclic or polycyclic ring system containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f};
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f};
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
X is selected from the group consisting of -S-, -S(O)-, and -S(O₂)-;
n is 0, 1, 2 or 3;
m is 0, 1, 2 or 3;
p is 0, 1, 2 or 3; and
q is 0, 1, 2 or 3.

The present invention also relates to a pharmaceutical composition comprising the compound of the present invention, or stereoisomers, tautomers, solvates, hydrates, active metabolites, isotopic labels, and pharmaceutically acceptable salts thereof.

The present invention also relates to use of the compound of the present invention, or stereoisomers, tautomers, solvates, hydrates, active metabolites, isotopic labels, and pharmaceutically acceptable salts thereof, and the pharmaceutical composition of the present invention in manufacture of pharmaceuticals for inhibiting TRPV3 activity.

The present invention also relates to use of the compound of the present invention, or stereoisomers, tautomers, solvates, hydrates, active metabolites, isotopic labels, and pharmaceutically acceptable salts thereof, and the pharmaceutical composition of the present invention in manufacture of pharmaceuticals for treating TRPV3-mediated conditions in a subject.

The present invention also relates to a method for treating TRPV3 mediated conditions, which comprises administering a therapeutically effective amount of the compound of the present invention, or stereoisomers, tautomers, solvates, hydrates, active metabolites, isotopic labels, and pharmaceutically acceptable salts thereof, or the pharmaceutical composition of the present invention to a patient in need of administration.

The thioether structure of the compound of the present invention has pharmacological activity, and after exerting pharmacological effects, it is rapidly converted into sulfoxide and sulfone metabolites that do not have pharmacological activity according to an established metabolic pathway, so that it can be achieved that the toxicity of prototype drugs, active metabolites and reactive metabolites to a body is avoided while exerting therapeutic effects, and the safety is relatively higher.

### Detailed Description

The present application will be further explained below in detail through embodiments. Through these descriptions, the features and advantages of the present application will become more clearly defined.

The term "exemplary" used here means "serving as an example, embodiment, or illustration." Any embodiment described here as "exemplary" should not be interpreted as being superior or better than other embodiments.

Furthermore, the technical features in different embodiments of the present application described below can be combined with each other if they do not conflict.

### Definitions

The terms "antagonist" and "inhibitor", which are used interchangeably, refer to pharmaceuticals that reduce or inhibit biological activity, for example inhibit the activity of ion channels such as TRPV3.

In terms of the present invention, for example, an "effective amount" of TRPV3 antagonist relates to the amount of the antagonist in the formulation that brings a desired clinical or functional result, when administered as part of a required dosage regimen. Without being bound by theory, an effective amount of a TRPV3 antagonist used in in the present invention comprises an amount of a TRPV3 antagonist effective to decrease one or more *in vitro* or *in vivo* functions of a TRPV3 channel. Exemplary functions comprises, but are not limited to, intracellular calcium levels, membrane polarization (e.g. an antagonists may promote cell hyperpolarization), Phase I outward current, Phase II outward current, Phase I inward current, and Phase II inward current. Compounds that antagonize TRPV3 function comprise compounds that antagonize an *in vitro* or *in vivo* functional activity of TRPV3. When a particular functional activity is only readily observable in an *in vitro* assay, the ability of a compound to inhibit TRPV3 function is a reasonable alternative for *in vitro* analysis of the activity of this compound. The term "preventing" is recognized in the art, and when used for a condition such as a local recurrence (e.g., pain), a disease such as cancer, a syndrome such as heart failure or any other medical condition, is well understood in the art, and comprises administration of a composition which reduces the frequency of, or delays the onset of, a medical condition in a subject relative to a subject which does not receive the composition. Thus, prevention of cancer comprises, for example, reducing the number of detectable cancerous growths in a population of patients receiving a prophylactic treatment relative to an untreated control population, and/or delaying the appearance of detectable cancerous growths in a treated population relative to an untreated control population, e.g., by a statistically and/or clinically significant amount. Prevention of an infection comprises, for example, reducing the number of diagnoses of the infection in a treated population relative to an untreated control population, and/or delaying the onset of conditions of the infection in a treated population relative to an untreated control population. Prevention of pain comprises, for example, reducing the magnitude of, or alternatively delaying, pain sensations experienced by subjects in a treated population relative to an untreated control population.

The present invention provides compounds in prodrug form. The term "prodrug" is intended to encompass compounds that, under physiological conditions, are converted into the therapeutically active agents of the present invention. A common method for preparing a prodrug comprises revealing the selected moieties of the required molecules after hydrolyzing under physiological conditions. In other embodiments, the prodrug is converted by enzymatic activity of a host animal. Additionally, prodrugs can be converted to the compounds of the present invention by chemical or biochemical methods in an ex vivo environment. For example, prodrugs can be slowly converted to the compounds of the present invention when placed in a transdermal patch reservoir with a suitable enzyme or chemical reagent.

The term 'oxidative metabolites' is intended to encompass compounds obtained by metabolizing parent compounds under normal physiological conditions. Specifically, an oxidative metabolite is formed by the oxidation of a parent compound during metabolism. For example, the oxidation of sulfide groups can generate the corresponding sulfoxides or sulfones.

The term "solvate" as used herein relates to compounds formed by solvation (for example, compounds formed by combining solvent molecules with solute molecules or ions).

The term 'hydrate' as used herein relates to compounds formed by the combination of water with parent compounds.

The term "treating" comprises prophylactic and/or therapeutic treatments. The term "prophylactic or therapeutic" treatment is recognized in the art, and comprises administering one or more compositions of the prssent invention to a host. If it is administered prior to clinical manifestation of a unwanted disease (e.g., diseases or other unwanted states of a host animal), the treatment is prophylactic (i.e., it protects the host against developing a unwanted condition), whereas if it is administered after manifestation of a unwanted condition, the treatment is therapeutic (i.e., it is intended to diminish, ameliorate, or stabilize the existing unwanted condition or side effects thereof).

The terms "TRPV3", "TRPV3 protein", and "TRPV3 channel" are used interchangeably throughout the present application. These terms refer to an ion channel (e.g., a polypeptide) comprising amino acid sequence, for example, the amino acid sequence of a human TRPV3 protein, or an equivalent polypeptide, or a functional bioactive fragment thereof. In certain embodiments, these term refer to those comprising, consisting of, or consisting essentially of, a TRPV3 amino acid sequence set forth, for example, in any patent application cited herein. TRPV3 protein may also comprise orthologs, for example, mouse, rat, horse, or Drosophila TRPV3.

TRPV3 comprises polypeptides that retain a function of TRPV3 and comprise (i) all or a portion of a TRPV3 amino acid sequence; (ii) a TRPV3 amino acid sequence with 1 to about 2, 3, 5, 7, 10, 15, 20, 30, 50, 75 or more conservative amino acid substitutions; (iii) an amino acid sequence that is at least 70%, 75%, 80%, 85%, 90%, 95%, 96%, 97%, 98%, or 99% identical to a TRPV3 amino acid sequence; and (iv) functional fragments thereof. Polypeptides of the present invention also comprise homologs, e.g., orthologs and paralogs, of a human TRPV3 polypeptide. TRPV3 polypeptides and amino acid sequences comprise, for example, the sequences set forth in any patent application cited herein.

The term " TRPV3" further relates to a nucleic acid encoding a polypeptide of the present invention, e.g., a nucleic acid comprising a sequence consisting of, or consisting essentially of, a TRPV3 polynucleotide sequence. A nucleic acid of the present invention may comprise all, or a portion of the following nucleotide sequences: (i) a TRPV3 nucleotide sequence; (ii) a nucleotide sequence that is at least 70%, 75%, 80%, 85%, 90%, 95%, 96%, 97%, 98% or 99% identical to a TRPV3 nucleotide sequence; (iii) a nucleotide sequence that hybridizes under stringent conditions to a TRPV3 nucleotide sequence; (iv) nucleotide sequences encoding polypeptides that are functionally equivalent to polypeptides of the invention; (v) nucleotide sequences encoding polypeptides having at least about 70%, 75%, 80%, 85%, 90%, 95%, 98%, 99% or more homology or identity with a TRPV3 polypeptide sequence; (vi) nucleotide sequences encoding polypeptides having the activity of a polypeptide of the present invention and having at least about 70%, 75%, 80%, 85%, 90%, 95%, 98%, 99% or more homology or identity with a TRPV3 polypeptide sequence; (vii) nucleotide sequences that differ by 1 to about 2, 3, 5, 7, 10, 15, 20, 30, 50, 75 or more nucleotide substitutions, additions or deletions of a TRPV3 nucleotide sequence, such as allelic variants; (viii) nucleic acids derived from and evolutionarily related to a TRPV3 nucleotide sequence; and (ix) complementary sequences for all of the aforementioned nucleic acids and other nucleic acids of the present invention, and nucleotide sequences resulting from the degeneracy of the genetic code. Nucleic acids of the invention also comprise homologs, e.g., orthologs and paralogs, of a TRPV3 nucleic acid sequence, and also variants which have been codon optimized for expression in a particular organism (e.g., host cell). TRPV3 nucleic acid sequences comprise, for example, the sequences set forth in any patent application cited herein. Where not explicitly stated, one of skill in the art can readily assess whether TRPV3 relates to a nucleic acid or a protein.

Herein, the term "aliphatic group" relates to a straight-chain, straight-chain, or cyclic aliphatic hydrocarbon group and comprises saturated and unsaturated aliphatic groups, such as an alkyl, an alkenyl, and an alkynyl.

The terms "alkenyl" and "alkynyl" refer to unsaturated aliphatic groups that are similar in length to the alkyls described above and may be substituted, but contain at least one double or triple bond respectively.

The terms "alkoxy" as used herein relates to an alkyl as defined below, to which an oxygen radical is attached. Representative alkoxyls comprise methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, -O-alkynyl, -O-(CH₂)ₜ-R₈, where R₈ is selected from the group consisting of hydrogen, halogen, lower alkyl, lower alkoxy, amino, or-NHSO₂NH₂ with t being an integer from 0 to 6.

The term "alkyl" relates to the radical of saturated aliphatic groups, comprising straight-chain alkyls and branched-chain alkyls. In preferred embodiments, a straight chain or branched chain alkyl has 30 or less, and more preferably 20 or less, and most preferably 10 or less carbon atoms in its backbone (e.g., C₁-C₃₀ for straight chains, C₃-C₃₀ for branched chains).

The term "cycloalkyl" comprises saturated and locally unsaturated cyclic alkyls with 3 to 12 carbons, preferably 3 to 8 carbons, and more preferably 3 to 6 carbons, wherein optionally, the cycloalkyl is additionally substituted. The preferred cycloalkyl has 3 to12 carbon atoms, and more preferably 5, 6, 7, or 8 carbon atoms in its ring structure. Preferred cycloalkyls comprise, but are not limited to, cyclopropyl, cyclobutyl, cyclopentyl, cyclopentenyl, cyclohexyl, cyclohexenyl, cycloheptyl, and cyclooctyl. The term "cycloalkyl" also comprises bridged ring groups, comprising but not limited to bicyclic [2.2.2] octane, bicyclic [1.1.1] pentane, bicyclic [3.2.1] octane, and bicyclic [2.1.1] hexane.

Moreover, the term "alkyl" (or "lower alkyl") as used throughout the specification, Examples, and claims is intended to comprise both "unsubstituted alkyls" and "substituted alkyls", the latter of which relates to alkyl moieties having substituents replacing hydrogens on one or more carbons of the hydrocarbon backbone. Such substituents can comprise, for example, a halogen, a hydroxyl, a carbonyl (such as a carboxyl, an alkoxycarbonyl, a formyl, or an acyl), a thiocarbonyl (such as a thioester, a thioacetate, or a thioformate), an alkoxyl, a phosphoryl, a phosphate, a phosphonate, a phosphonite, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or aromatic or heteroaromatic moiety. It will be understood by those skilled in the art that the moieties substituted on the hydrocarbon chain can themselves be substituted, if appropriate. For instance, the substituents of a substituted alkyl may comprise substituted and unsubstituted forms of amino, azido, imino, amido, phosphoryl (comprsing phosphonate and phosphonite), sulfonyl (comprsing sulfate, sulfonamido, sulfamoyl and sulfonate), and silyl, as well as ethers, alkylthios, carbonyls (comprsing ketones, aldehydes, carboxylates, and esters), -CF₃, -CN and the like. Exemplary substituted alkyls are described below. A cycloalkyl can be further substituted with alkyl, alkenyl, alkoxy, alkylthio, aminoalkyl, carbonyl-substituted alkyl, -CF₃, -CN, and the like.

Analogous substitutions can be made to alkenyl and alkynyls to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

Unless the number of carbons is otherwise specified, "lower alkyl" as used herein means an alkyl as defined above, but having 1 to 10 carbons, more preferably 1 to 6 carbon atoms in its backbone structure. Likewise, "lower alkenyl" and "lower alkynyl" have similar chain lengths. Throughout the application, preferred alkyls are lower alkyls. In preferred embodiments, a substituent known as alkyl herein is a lower alkyl.

The term "alkylthio" relates to an alkyl as defined above, to which a sulfur radical is attached. In preferred embodiments, the "alkylthio" moiety is represented by one of -S-alkyl, - S-alkenyl, -S-alkynyl, and -S-(CH₂)ₜ-R₈ wherein t and R₈ are defined as above. Representative alkylthios comprise methylthio, ethylthio, and the like.

The term "aralkyl", as used herein, relates to an alkyl substituted with an aryl (e.g., an aromatic or heteroaromatic group).

The term "aryl" as used herein comprises 5-, 6-, and 7-membered single-ring aromatic groups that may comprise 0 to 4 heteroatoms, for example, benzene, pyrrole, furan, thiophene, imidazole, oxazole, thiazole, triazole, pyrazole, pyridine, pyrazine, pyridazine and pyrimidine, and the like. Those aryls having heteroatoms in the ring structure may also be referred to as "aryl heterocycles" or "heteroaromatics." The aromatic ring can be substituted at one or more ring positions with such substituents as described above, for example, halogen, azide, alkyl, aralkyl, alkenyl, alkynyl, cycloalkyl, hydroxyl, alkoxyl, amino, nitro, sulfhydryl, imino, amido, phosphate, phosphonate, phosphonite, carbonyl, carboxyl, silyl, ether, alkylthio, sulfonyl, sulfonamido, ketone, aldehyde, ester, heterocyclyl, aromatic or heteroaromatic moieties, -CF₃, - CN, or the like. The term "aryl" also comprises polycyclic ring systems having two or more rings in which two or more carbons are common to two adjoining rings (the rings are "fused rings"), wherein at least one of the rings is aromatic, e.g., the other cyclic rings can be cycloalkyls, cycloalkenyls, cycloalkynyls, aryls and/or heterocyclyls.

The term "carbocycle" as used herein relates to an aromatic or non-aromatic ring, in which each atom of the ring is carbon.

The term "electron withdrawing group" relates to a chemical group that attracts electron density from the atoms or the atomic groups bound with electron withdrawing groups. Attracting electron density comprises attraction through induction effect or delocalization/resonance effect. Examples of electron withdrawing groups connected to aromatic rings comprise perfluoroalkyls such as trifluoromethyl, halogens, azides, carbonyl-containing groups such as acyl, cyano, and imine-containing groups.

The term "heteroatom" as used herein means an atom of any element other than carbon or hydrogen. Preferred heteroatoms are boron, nitrogen, oxygen, phosphorus, sulfur and selenium.

The terms "heterocyclyl" or "heterocyclic group" refer to 3- to 10-membered ring structures, more preferably 3- to 7-membered rings, whose ring structures comprise 1 to 4 heteroatoms. Heterocycles can also be polycycles. Heterocyclyls comprise, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, for example, halogen, alkyl, aralkyl, alkenyl, alkynyl, cycloalkyl, hydroxyl, amino, nitro, sulfhydryl, imino, amido, phosphate, phosphonate, phosphonite, carbonyl, carboxyl, silyl, ether, alkylthio, sulfonyl, ketone, aldehyde, ester, heterocyclyl, aromatic or heteroaromatic moiety, -CF₃, -CN, or the like.

The term "nitro" as used herein means -NO₂; the term "halogen" means -F, -Cl, -Br or -I; the term "sulfhydryl" means -SH; the term "hydroxyl" means -OH; and the term "sulfonyl" means -SO₂-.

The terms "polycyclyl", "polycyclic group" or "polycyclic system" refer to two or more rings (e.g., cycloalkyl, cycloalkenyl, cycloalkynyl, aryls and/or heterocyclyl), in which one, two or more carbons are common to two adjoining rings, for example, the rings are "fused rings" or " spiro ring". Rings that are joined through non-adjacent atoms are termed "bridged" rings, for example C₅-C₁₂ bridged carbon ring, comprsing but not limited to bicyclic [2.2.2] octane, bicyclic [1.1.1] pentane, bicyclic [3.2.1] octane, and bicyclic [2.1.1] hexane. Each ring in the polycycle can be substituted with such substituents as described above, for example, halogen, alkyl, aralkyl, alkenyl, alkynyl, cycloalkyl, hydroxyl, amino, nitro, sulfhydryl, imino, amido, phosphate, phosphonate, phosphonite, carbonyl, carboxyl, silyl, ether, alkylthio, sulfonyl, ketone, aldehyde, ester, heterocyclyl, aromatic or heteroaromatic moiety, -CF₃, -CN, or the like.

The term "protecting group" as used herein means temporary substituents which protect a potentially reactive functional group from undesired chemical transformations. Examples of such protecting groups comprise esters of carboxylic acids, silyl ethers of alcohols, and acetals and ketals of aldehydes and ketones, respectively. The protecting groups in chemistry field have been reviewed (Greene, T.W.; Wuts, P.G.M. Protective Groups in Organic Synthesis, 2nd ed.; Wiley: New York, 1991).

The term "substituted" as used herein is contemplated to comprise all permissible substituents of organic compounds. In a broad aspect, the permissible substituents comprise acyclic and cyclic, branched and unbranched, carbocyclic and heterocyclic, aromatic and nonaromatic substituents of organic compounds (e.g., alkyl, alkenyl, alkynyl, cycloalkyl, heterocyclyl, aryl, heteroaryl, cycloalkylalkyl, heterocyclylalkyl, aralkyl, or heteroaralkyl, wherein any substituent itself may be further substituted), as well as halogen, carbonyl (e.g., ester, carboxyl, or formyl), thiocarbonyl (e.g., thioester, thiocarboxylate, or thioformate), ketone, aldehyde, amino, acylamino, amido, amidino, cyano, nitro, azido, sulfonyl, sulfoxido, sulfate, sulfonate, sulfamoyl, sulfonamido, and phosphoryl. Illustrative substituents comprise, for example, those described above. The permissible substituents can be one or more and the same or different for appropriate organic compounds. For purposes of the present invention, a heteroatom such as nitrogen may have hydrogen substituents and/or any permissible substituents of organic compounds described herein, which satisfy the valence of the heteroatom. The present invention is not intended to be limited in any manner by the permissible substituents of organic compounds.

It will be understood that "substitution" or "substituted with" comprises the implicit proviso that such substitution is in accordance with permitted valence of the substituted atom and the substituent, and that the substitution results in a stable compound, e.g., which does not spontaneously undergo transformation such as by rearrangement, cyclization, elimination, etc. The phrase "substituted with 0 to 2 R^{f}" means that the corresponding group carry 0, 1, or 2 R^{f}.

As used herein, the definition for each expression, when it occurs more than once in any structure, is intended to represent an independent definition unless it is in the same structure.

The abbreviations Me, Et, Ph, Tf, Nf, Ts, Ms represent methyl, ethyl, phenyl, trifluoromethanesulfonyl, nonafluorobutanesulfonyl, *p*-toluenesulfonyl and methanesulfonyl, respectively. A more comprehensive list of the abbreviations utilized by an ordinary skill in the field of organic chemistry appears in the first issue of each volume of the *Journal of Organic Chemistry;* this list is typically presented in a table entitled Standard List of Abbreviations. The abbreviations contained in said list, and all abbreviations utilized by an ordinary skill in the field of organic chemistry are hereby incorporated by reference.

Certain compounds of the present invention may exist in particular geometric or stereoisomeric forms. The present invention contemplates that all such compounds, including cis- and trans-isomers, *R*- and *S*-enantiomers, diastereomers, (D)-isomers, (L)-isomers, the racemic mixtures thereof, and other mixtures thereof, fall within the scope of the present invention. Additional asymmetric carbon atoms may be present in a substituent such as an alkyl. All such isomers, as well as mixtures thereof, are intended to be comprised in the present invention.

Methods for preparing substantially isomerically pure compounds are known in the art. If, for instance, a particular enantiomer of the compound of the present invention is desired, it may be prepared by asymmetric synthesis, or by derivation with a chiral auxiliary, where the resulting diastereomeric mixture is separated and the auxiliary group is removed in order to provide the desired pure enantioniers. Alternatively, where the molecule contains a basic functional group, such as amino, or an acidic functional group, such as carboxyl, diastereomeric salts may be formed with an appropriate optically active acid or base, and then the resulting diastereomer is separated by well-known fractional crystallization or chromatographic methods in the field, and the pure enantiomer is subsequently recovered. Alternatively, enantiomerically enriched mixtures and pure enantiomeric compounds can be prepared by using synthetic intermediates that are enantiomerically pure in combination with reactions that either maintain the stereochemistry at a chiral center unchanged or result in its complete inversion. Techniques for inverting a particular stereocenter or maintaining it unchanged, and those for seprating mixtures of stereoisomers are well known in the art, and it is well within the ability of one of skill in the art to choose an appropriate method for a particular situation. See, generally, Furniss et al. (eds.), Vogel's Encyclopedia of Practical Organic Chemistry 5th Ed., Longman Scientific and Technical Ltd., Essex, 1991, pp. 809-816; and Heller, Acc. Chem. Res. 23: 128 (1990).

Contemplated equivalents of the compounds described above comprise the compounds which otherwise correspond thereto, and which have the same general properties thereof (e.g., the ability to inhibit TRPV3 activity), wherein it is ensured that one or more simple variants of substituents do not adversely affect the efficacy of the compound. In general, the compounds of the present invention are prepared by, for example, the methods illustrated in the general reaction schemes described below, or by modifications thereof, using readily available starting materials, reagents and conventional synthesis procedures. In these reactions, it is also possible to make use of variants that are already known, but are not mentioned here.

For purposes of the present invention, the chemical elements are identified in accordance with the Periodic Table of the Elements. Also for purposes of the present invention, the term "hydrocarbon" is contemplated to comprise all permissible compounds having at least one hydrogen and one carbon atom. In a broad aspect, the permissible hydrocarbons comprise acyclic and cyclic, branched and unbranched, carbocyclic and heterocyclic, aromatic and nonaromatic organic compounds which can be substituted or unsubstituted.

The compounds of the present invention may also contain unnatural proportions of atomic isotopes at one or more atoms that constitute such compounds. For example, the compounds may be radiolabeled with radioactive isotopes, such as deuterium (²H), tritium (³H), iodine-125 (¹²⁵I) or carbon-14 (¹⁴C). All isotopic variants of the compounds of the present invention, whether radioactive or not, are intended to be encompassed within the scope of the present invention.

The symbol " 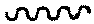 " , whether utilized as a bond or shown perpendicular to a bond, indicates the point at which the displayed moiety is attached to the remainder of the molecule, solid support, etc.

Certain compounds of the present invention can exist in unsolvated forms as well as solvated forms, comprsing hydrated forms. In general, the solvated forms are equivalent to unsolvated forms and are encompassed within the scope of the present invention. Certain compounds of the present invention may exist in polycrystalline or amorphous forms. In general, all physical forms are equivalent for the uses contemplated by the present invention and are intended to be within the scope of the present invention.

Substituent groups are specified by their conventional chemical formulae, written from left to right, and they equally encompass the chemically identical substituents, which would result from writing the structure from right to left, for example, -CH₂O- is also intended to represent -OCH₂-; -NHS(O)₂- is also intended to represent -S(O)₂HN-; etc.

The term "pharmaceutically acceptable salts" comprises salts of the active compounds which are prepared with relatively nontoxic acids or bases, depending on the particular substituents found on the compounds described herein. When compounds of the present invention contain relatively acidic functionalities, base addition salts can be obtained by contacting the neutral form of such compounds with a sufficient amount of the desired base, either purely or in a suitable inert solvent. Examples of pharmaceutically acceptable base addition salts comprise sodium, potassium, calcium, ammonium, organic amino, or magnesium salts, or similar salts. When compounds of the present invention contain relatively basic functionalities, acid addition salts can be obtained by contacting the neutral form of such compounds with a sufficient amount of the desired acid, either purely or in a suitable inert solvent. Examples of pharmaceutically acceptable acid addition salts comprise those derived from inorganic acids such as hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from relatively nontoxic organic acids such as acetic, trifluoroacetic, propionic, isobutyric, maleic, malonic, benzoic, succinic, suberic, fumaric, lactic, mandelic, phthalic, benzensulfonic, p-tolylsulfonic, citric, tartaric, methanesulfonic, and the like. Also comprised are the salts of amino acids such as arginate and the like, and salts of organic acids such as glucuronic or galactunoric acids and the like (see, for example, Berge et al., "Pharmaceutical Salts", Journal of Pharmaceutical Science, 1977, 66, 1-19). Certain specific compounds of the present invention contain both basic and acidic functionalities, so that the compounds can be converted into either base or acid addition salts.

The neutral forms of the compounds are preferably regenerated by contacting the salt with a base or acid and isolating the parent compound in the conventional manner. The parent form of the compound differs from the various salt forms in certain physical properties, such as solubility in polar solvents, but otherwise the salts are equivalent to the parent form of the compound for the purposes of the present invention.

The term "low enough pyrogen activity", with reference to a pharmaceutical preparation, means that the content of a pyrogen in a preparation would not lead to a side effect (e.g., irritation, fever, inflammation, diarrhea, respiratory distress, endotoxic shock, etc.) in a subject to which the preparation has been administered. For example, the term encompasses preparations that are free of, or substantially free of, an endotoxin, for example, a lipopolysaccharide (LPS).

### Diseases, Disorders, or Conditions Related to TRPV3 Function

In an embodiment of the methods for preventing or treating a disease or disorder or condition, the pharmaceutical being administered is one that modulates the level and/or activity of a TRPV3 protein. In certain embodiments, the compound inhibits the expression and/or activity of a TRPV3 protein. In other embodiments, the compound selectively inhibits the expression of a TRPV3 protein. In other words, in certain embodiment, compared to one or more other ion channels, the compound preferentially inhibits the activity of a TRPV3 protein.

In particular embodiments of the methods for preventing or treating diseases and disorders provided herein, the disease or disorder can be, for example, a pain or sensitivity to touch such as pain related to a disease or disorder, e.g., cancer pain, a dermatological disease or disorder, e.g., psoriasis as well as basal cell carcinomas and squamous cell cariconomas, a neurodegenerative disease or disorder, e.g., Alzheimer's disease (AD), Parkinson's disease, Huntington's disease, amyotrophic lateral sclerosis (ALS), and other brain diseases caused by trauma or other insults including aging, an inflammatory disease (e.g., asthma, chronic obstructive pulmonary disease, rheumatoid arthritis, osteoarthritis, inflammatory bowel disease, glomerulonephritis, neuroinflammatory diseases, multiple sclerosis, and disorders of the immune system), cancer or other proliferative disease, kidney disease and liver disease, metabolic disorder such as diabetes. Further diseases and conditions comprise post-surgical pain, post herpetic neuraligia, fibromyalgia, and shingles.

Because of the important role that calcium regulation plays in many cellular processes including cellular activation, gene expression, cellular trafficking and apoptotic cell death, calcium dyshomeostasis is implicated in the many diseases and disorders involving such cellular activities. These diseases and disorders comprise dermatological diseases and disorders; neurological and neurodegenerative diseases and disorders; fever associated with various diseases, disorders or conditions; incontinence; inflammatory diseases and disorders such as inflammatory bowel disease and Crohn's disease; respiratory diseases and disorders such as chronic cough, asthma and chronic obstructive pulmonary disease (COPD); digestive disorders such as ulcers and acid reflux; metabolic diseases and disorders including obesity and diabetes; liver and kidney diseases and disorders; malignancies including cancers; aging-related disorders; and sensitivity to pain and touch.

Other diseases or conditions that can be treated comprise ATP related diseases or disorders, including epilepsy, cognition, vomiting, pain (such as migraine), asthma, peripheral vascular diseases, hypertension, immune and inflammatory conditions, irritable bowel syndrome, cystitis, depression, age-related degenerative diseases, urinary incontinence, premature ejaculation, cystic fibrosis, diabetes, birth control and infertility, and wound healing (for example, see Foresta et al. (1992) J. Biol. Chem. 257: 19443-19447; Wang et al. (1990)Biochim.Biophys.Res. Commun. 166: 251-258; Burnstock and Williams, (2000)J.Pharmacol.Exp.Ther.295: 862-869; and Burnstock, Pharmacol Rev(2006)58: 58-86).

The TRPV3 inhibitors described herein can be used to treat any of the aforementioned and subsequent diseases or conditions, including the treatment of pain associated with any of the aforementioned or subsequent diseases or conditions. When used in treatment methods, inhibitors can be selected and formulated based on predetermined routes of administration.

Compositions and methods provided herein can be used in the prevention or treatment of pain or sensitivity to pain and touch. Pain or sensitivity to pain and touch may be indicated in a variety of diseases, disorders or conditions, comprising, but not limited to, diabetic neuropathy, breast pain, psoriasis, eczema, dermatitis, bum, post-herpetic neuralgia (shingles), nociceptive pain, peripheral neuropathic and central neuropathic pain, chronic pain, cancer and tumor pain, spinal cord injury, crush injury and trauma induced pain, migraine, cerebrovascular and vascular pain, Sickle cell disease pain, rheumatoid arthritis pain, musculoskeletal pain including treating signs and symptoms of osteoarthritis and rheumatoid arthritis, orofacial and facial pain including dental- and cancer-related lower back or pelvic pain, surgical incision related pain, inflammatory and non-inflammatory pain, visceral pain, psychogenic pain and soft tissue inflammatory pain, fibromyalgia-related pain, and reflex sympathetic dystrophy. The compounds and methods of the present invention can be used in the treatment of chronic as well as acute pain. Chronic or acute pain may be the result of injury, age, or disease.

Other ion channels have been implicated in reception or transmission of pain. For example, the involvement of N-type calcium channels in the synaptic transmissions that convey pain signals from sensory afferent nerve cells to the central nervous system has been recognized. Certain naturally occurring peptide neurotoxins that specifically block N-type calcium channel have been shown to act as extremely potent and efficient analgesics in a wide range of animal pain models, which comprise models of inflammatory and neuropathic pain. The available evidence suggests that N-type calcium channel blockers are at least as effective as opiates, are devoid of a number of the typical opiate side effects (e.g. respiratory depression) and that the analgesic effect is not subject to tolerance development.

TRPV3, as well as TRPV1 and TRPV4, are expressed in a pattern consistent with involvement in pain. TRPV3 is expressed in pain sensitive neurons, and this expression is upregulated following injury. In addition, TRPV3 is robustly expressed in skin. Accordingly, methods for treating pain comprise administration of (i) antagonists of a TRPV3 function; (ii) combinations of selective antagonists of a TRPV3 and TRPV1 and/or TRPV4 function; or (iii) a pan-TRP inhibitor that inhibits functions of TRPV3, TRPV1, and TRPV4.

In addition to TRPV family members, other TRP channels have been implicated in pain reception and/or sensation. For example, certain TRPM channels including TRPM8 have been implicated in the reception and/or sensation of pain. Accordingly, in certain embodiments, the methods of the present invention comprise treating pain by administering (i) a combination of a selective TRPV3 antagonist and a selective TRPM8 antagonist; (ii) a combination of a selective TRPV3 antagonist, a selective TRPM8 antagonist, and one or more of a selective TRPV1 and/or TRPV4 antagonist; (iii) a cross-TRP inhibitor that antagonizes a function of TRPV3 and TRPM8; or (iv) a pan inhibitor that antagonizes a function of TRPV3, TRPM8, and one or more of TRPV1 and TRPV4.

Influx of calcium across plasma membrane of skin cells is a critical signaling element involved in cellular differentiation in the skin epidermis (Dotto, 1999 Crit Rev Oral Biol Med 10:442-457). Regulating or modulating the calcium entry pathway, and thus a critical control point for skin cell growth, can treat or prevent skin diseases or disorders that are characterized by epidermal hyperplasia, i.e., a condition in which skin cells both proliferate too rapidly and differentiate poorly. Such diseases comprise psoriasis, basal cell carcinomas and squamous cell carcinomas. Psoriasis, estimated to affect up to 7 million Americans, afflicts sufferers with mild to extreme discomfort, enhanced susceptibility to secondary infections, and psychological impact due to disfigurement of the affected areas (Lebwohl and Ali, 2001. J Am Acad Dermatol 45:487-498). Basal cell carcinomas (BCC) and squamous cell carcinomas (SCC) of the skin represent at least one-third of all cancers diagnosed in the United States each year. More than 1 million new cases are reported annually and incidence is increasing. Despite being relatively non-aggressive, slow-growing cancers, BCCs can result in significant local tissue destruction and disfigurement. SCCs are more aggressive and thus present even greater complications. Moreover, given that 80% of lesions are on the head and neck with another 15% on shoulders, back or chest, BCCs and SCCs of the skin can have a significant impact on the appearance and quality of life of the afflicted patient.

Many dermatological disorders are accompanied by itch (pruritus). Pruritus and pain share many mechanistic similarities. Both are associated with activation of C-fibers, both are potentiated by increases in temperature and inflammatory mediators and both can be quelled with opiates. Decreasing neuronal excitability, particularly C-fiber excitability can alleviate pruritus associated with dialysis, dermatitis, pregnancy, poison ivy, allergy, dry skin, chemotherapy and eczema.

Acne is a dermatological disorder of complex etiology. Among other factors, secretion of oils from the sebaceous glands contributes to the development of acne. Since TRPV3 is also expressed in the sebaceous gland and has been shown to be able to regulate secretion in other skin cells, antagonizing TRPV3 function may reduce the signs and symptoms of acne.

In certain preferred embodiments, the TRPV3 antagonist is administered to prevent, treat, or improve signs and symptoms of acute pain, chronic pain, touch sensitivity, itch sensitivity, or as part of the treatment of burns, such as post-surgical pain, cancer pain, or neuropathic pain.

In certain preferred embodiments, the TRPV3 antagonist is administered to prevent, treat, or improve signs and symptoms of migraine.

In certain preferred embodiments, the TRPV3 antagonist is administered to prevent, treat, or improve signs and symptoms of disorders or conditions which are selected from diabetic neuropathy, inflammation, psoriasis, eczema, dermatitis, post-herpetic neuralgia (shingles), incontinence, bladder incontinence, fever, hot flashes, and cough.

In certain preferred embodiments, the TRPV3 antagonist is administered to prevent, treat, or improve signs and symptoms of osteoarthritis.

In certain preferred embodiments, the TRPV3 antagonist is administered to prevent, treat, or improve signs and symptoms of rheumatoid arthritis.

In certain preferred embodiments, the TRPV3 antagonist is administered to prevent, treat, or improve signs and symptoms of Oral Mucositis.

In certain preferred embodiments, the TRPV3 antagonist is administered to promote hair loss or inhibit hair growth in patients.

Another aspect of the present invention relates to the use of TRPV3 antagonists in manufacture of pharmaceuticals that prevent, treat, or improve symptoms of diseases, disorders or conditions in patients, wherein the symptoms involve the activation of TRPV3, or for the symptoms, a reduced TRPV3 activity can reduce severity.

### Pharmaceutical Composition

While the compound of the present invention can be administered alone, it is preferable to administer the compound as a pharmaceutical formulation (composition). The compounds according to the invention can be formulated for administration in any convenient way for medicinal use in human or veterinary. In certain embodiments, the compound comprised in the pharmaceutical formulation can be active itself, or can be a prodrug which, for example, is capable of being converted to an active compound in a physiological setting.

Regardless of the route of administration selected, the compounds of the present invention, which is used in a suitable hydrated form, and/or the pharmaceutical compositions of the present invention, maybe formulated into the following pharmaceutically acceptable dosage forms by other conventional methods known to those of skill in the art.

Thus, another aspect of the present invention provides a pharmaceutically acceptable composition comprising a therapeutically effective amount of one or more compounds described above, formulated together with one or more pharmaceutically acceptable carriers (additives) and/or diluents into a formulation. As described in detail below, the pharmaceutical composition of the present invention can be specially formulated for administration in solid or liquid form, comprsing those adapted for the following: (1) oral administration, for example, drenches (aqueous or non-aqueous solutions or suspensions); tablets; boluses; powders; granules; pastes for application to the tongue, teeth, lips, gums; mouth washes; gels; (2) parenteral administration, for example, as a sterile solution or suspension, by subcutaneous, intramuscular or intravenous injection; (3) topical application, for example, as a cream, ointment or spray applied to skin; (4) intravaginally or intrarectally, for example, as a vaginal suppository, cream or foam; or (5) for inhalation. However, in certain embodiments, the compounds of the present invention can be easily dissolved or suspended in sterile water. In certain embodiments, the pharmaceutical formulation is non-pyrogenic, i.e., does not elevate the body temperature of a patient.

The TRPV3 antagonist can be administered alone or in combination with other therapeutic agents. For example, the TRPV3 antagonist is administered in combination with one or more of the following therapeutic agents: anti-inflammatory agents, anti-acne agents, anti-wrinkle agents, anti-scarring agent, anti-psoriatic agents, anti-proliferative agents, anti-fungal agents, anti-viral agents, preservatives, anti-migraine agents, keratolytic agents, or hair growth inhibitors.

The TRPV3 antagonist can be administered locally, orally, percutaneously, rectally, vaginally, parenterally, intranasally, intraocularly, intravenously, intramuscularly, intraarterially, intrathecally, intracapsularly, intraorbitally, intracardiacly, intradermally, intraperitoneally, transtracheally, subcutaneously, subcuticularly, intraarticularly, subcapsularly, subarachnoidly, intraspinally, and intrasternally, or by inhalation.

In certain preferred embodiments, the TRPV3 antagonist is administered locally.

In certain preferred embodiments, the TRPV3 antagonist is administered orally.

In certain preferred embodiments, the TRPV3 antagonist is administered parenterally.

The term "therapeutically effective amount" as used herein means that amount of a compound, material, or composition comprising a compound of the present invention which is effective for producing some desired therapeutic effect by inhibiting TRPV3 function in at least a sub-population of animal cells and thereby blocking the biological consequences of that function in the treated cells, at a reasonable benefit/rish ratio applicable to any medical treatment.

The terms "systemic administration," "administered systemically," "peripheral administration" and "administered peripherally" as used herein mean that a compound, pharmaceutical or other material are not administered directly into the central nervous system, so that it enters the patient's system, and thus, is subject to metabolism and other processes, for example, subcutaneous administration.

The term "pharmaceutically acceptable" is employed herein to refer to those compounds, materials, compositions, and/or dosage forms which are, within the reasonable scope of medical evaluation, suitable for use in contact with the tissues of human beings and animals without excessive toxicity, irritation, allergic response, or other problem or complication, and commensurate with a reasonable benefit/risk ratio.

The term "pharmaceutically acceptable carrier" as used herein means a pharmaceutically acceptable material, composition or vehicle, such as a liquid or solid filler, diluent, excipient, solvent or encapsulating material, which participate in carrying or transporting the antagonist of the present invention from one organ or part of the body to another. Each carrier must be "acceptable" in the sense of being compatible with the other ingredients of the formulation and not injurious to the patient. Some Examples of materials which can serve as pharmaceutically acceptable carriers comprise: (1) sugars, such as lactose, glucose and sucrose; (2) starches, such as corn starch and potato starch; (3) cellulose and derivatives thereof, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethanol; (20) phosphate buffer solutions; and (21) other non-toxic compatible substances employed in pharmaceutical formulations.

As mentioned above, certain embodiments of the present compounds can contain a basic functional group, such as amino or alkylamino, and thus, are capable of forming pharmaceutically acceptable salts with pharmaceutically acceptable acids. In this respect, the term "pharmaceutically acceptable salts" relates to the relatively non-toxic, inorganic and organic acid addition salts of the compounds of the present invention. These salts can be prepared *in situ* during the final separation and purification of the compounds of the invention, or by separately reacting the purified compound of the present invention in its free base form with a suitable organic or inorganic acid, and seprating the salt thus formed. Representative salts comprise the hydrobronide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like (See, for example, Berge et al. (1977) "Pharmaceutical Salts", J. Pharm. Sci. 66:1-19).

The pharmaceutically acceptable salts of the compounds of the present invention comprise the conventional nontoxic salts or quaternary ammonium salts of the compounds, e.g., from non-toxic organic or inorganic acids. For example, such conventional nontoxic salts comprise those derived from inorganic acids such as hydrochloride, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, palmitic, maleic, hydroxymaleic, phenylacetic, glutamic, benzoic, salicyclic, sulfanilic, 2-acetoxybenzoic, fumaric, toluenesulfonic, methanesulfonic, ethane disulfonic, oxalic, isothionic, and the like.

In other cases, the compounds of the present invention can contain one or more acidic functional groups, and thus, are capable of forming pharmaceutically acceptable salts with pharmaceutically acceptable bases. The term "pharmaceutically acceptable salts" in these instances relates to the relatively non-toxic, inorganic and organic base addition salts of the compounds of the present invention. These salts can likewise be prepared *in situ* during the final separation and purification of the compounds, or by separately reacting the purified compound in its free acid form with a suitable base, such as the hydroxide, carbonate or bicarbonate of a pharmaceutically acceptable metal cation; with ammonia; or with a pharmaceutically acceptable organic primary, secondary or tertiary amine. Representative alkali or alkaline earth salts comprise lithium, sodium, potassium, calcium, magnesium, and aluminum salts and the like. Representative organic amines useful for the formation of base addition salts comprise ethylamine, diethylamine, ethylenediamine, ethanolamine, diethanolamine, piperazine and the like.

Examples of pharmaceutically acceptable antioxidants comprise: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention comprise those suitable for oral, nasal, local (including buccal and sublingual), rectal, vaginal and/or parenteral administration. Oral formulations comprise those delivered to and maintained in the mouth without swallowing, as well as formulations that are swallowed as part of or following use. The formulations can conveniently be presented in unit dosage form and can be prepared by any methods well known in the art of pharmacy. The amount of active ingredient which can be combined with a carrier material to produce a single dosage form will vary depending upon the host being treated, the particular mode of administration. The amount of active ingredient that can be combined with a carrier material to produce a single dosage form will generally be that amount of the compound which produces a therapeutic effect. Generally, based on 100%, this amount will range from about 1% to about 99% of active ingredient, preferably from about 5% to about 70%, most preferably from about 10% to about 30%.

Methods of preparing these formulations or compositions comprise the step of combining a compound of the present invention with the carrier and optionally one or more accessory ingredients. In general, the formulations are prepared by uniformly and intimately combining a compound of the present invention with liquid carriers, or finely divided solid carriers, or both, and then, if necessary, shaping the product.

Formulations of the present invention suitable for oral administration can be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of the compound of the present invention as active ingredient. The compound of the present invention can also be administered as a bolus, electuary or paste.

The present invention relates to a compound of formula (I), or stereoisomers, tautomers, solvates, hydrates, active metabolites, isotopic labels, and pharmaceutically acceptable salts thereof: 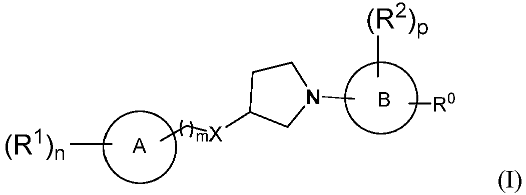
wherein ring A and ring B are each independently selected from a monocyclic or polycyclic ring system containing 3-12 ring atoms;
R¹ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}), -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R², together with the atoms of ring B to which they are connected, form a 3-10 membered ring structure;
R⁰ is each independently selected from the group consisting of H, halogen or a structure of formula II 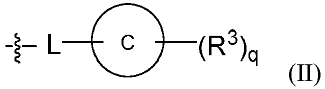
wherein,
L is each independently selected from the group consisting of a bond, -O-, -S-, -N(R²⁰)-, - C(O)-, -C(R²⁰R²¹)-, -S(O)- and -S(O₂)-;
ring C is each independently selected from monocyclic or polycyclic ring systems containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f};
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f};
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
X is selected from the group consisting of -S-, -S(O)-, and -S(O₂)-;
n is 0, 1, 2 or 3;
m is 0, 1, 2 or 3;
p is 0, 1, 2 or 3;
q is 0, 1, 2 or 3.

In one embodiment, the compound has a structure of formula I-1 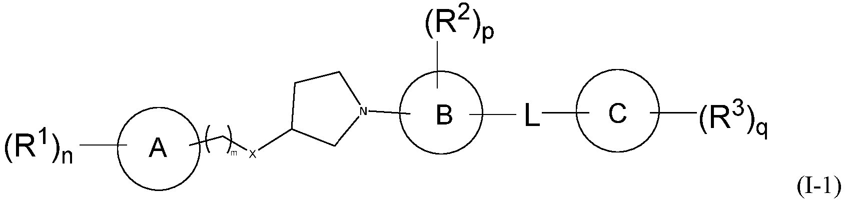 wherein ring A, ring B, ring C, X, R¹, R², R³, L, n, p, m, and q are defined as formula I.

In one embodiment, ring A is selected from the group consisting of benzene ring, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

In one embodiment, ring A is selected from the following structural formulae:

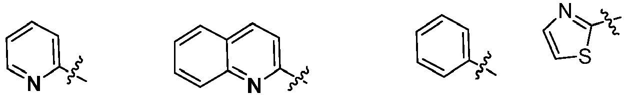

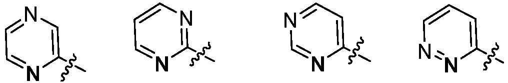

In one embodiment, ring B is selected from the group consisting of benzene ring, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

In one embodiment, ring B is selected from the following structural formulae:

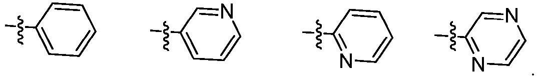

In one embodiment, R¹ is each independently selected from the group consisting of H, halogen, cyano, hydroxyl, C₁-C₆ alkyl, C₁-C₆ haloalkyl, C₁-C₆ alkoxy, -N(R^{a})(R^{b}) and -R¹¹OR¹², wherein R^{a} and R^{b} are each independently selected from the group consisting of H and C₁-C₆ alkyl; R¹¹ is each independently selected from C₁-C₆ alkylene; R¹² is each independently selected from the group consisting of H, and C₁-C₆ alkyl.

In one embodiment, R¹ is each independently selected from the group consisting of H, Cl, F, -CF₃, -CN, -CH₃, -OH, -OCH₃, and -CH₂OCH₃.

In one embodiment, n is 0, 1 or 2.

In one embodiment, m is 0, that is, X is directly connected to ring A.

In one embodiment, when n is 1 and m is 0, on ring A, R¹ is located at the ortho or para position of X.

In one embodiment, when n is 2 and m is 0, on ring A, one R¹ is located at the para position of X.

In one embodiment, R² is each independently selected from the group consisting of H, cyano, hydroxyl, C₁-C₆ alkyl, C₁-C₆ haloalkyl, C₃-C₆ cycloalkyl substituted with 0 to 2 R^{f}, C₁-C₆ alkoxy, C₁-C₆ haloalkoxy, -R¹¹OR¹², -R¹¹SR¹², -C(O)OR^{d} and -C(O)N(R^{a})(R^{b}); wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{d} is each independently selected from the group consisting of H and C₁-C₆ alkyl; R^{f} is each independently selected from the group consisting of halogen, hydroxyl, amino and C₁-C₆ alkyl.

In one embodiment, R² is each independently selected from the group consisting of H, - CH₃, -OH, -CH₂OH, -CH₂OCH₃, -CH₂CH₂OH, -CH(CH₃)OH, -C(CH₃)₂OH, -CH₂SH, - OCH₂CH₃, -OCF₃, -OCH₃, -OCH(CH₃)₂, -COOH, -COOCH₃, -CONH₂, -CH₂NH₂, -CH₂CHF₂, -CN, -CHF₂, 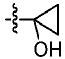 and 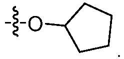 Preferably, R² is -CH₂OH.

In one embodiment, p is 1, that is, only one R² as defined above is substituted on ring B. In the embodiment, in which p is 1, the R² is located at the ortho position of pyrrolidinyl.

In one embodiment, L is each independently selected from the group consisting of a bond, -O-, -S-, -N-, -C(O)-, -CH₂-, -C(OH)-, -S(O)-, and -S(O₂)-.

In one embodiment, L is -S-, or L is -S(O)-, or L is -S(O₂)-. Preferably, L is -S-.

In one embodiment, ring C is selected from the group consisting of C₃-C₆ cycloalkane ring, benzene ring, benzo-C₃-C₆ cycloalkane rings, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

In one embodiment, ring C is selected from the following structural formulae:

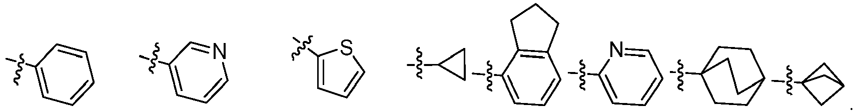

In one embodiment, R³ is each independently selected from the group consisting of H, cyano, hydroxyl, halogen, C₁-C₆ alkyl, C₁-C₆ haloalkyl, C₁-C₆ alkoxy, C₁-C₆ haloalkoxy, C₃-C₆ cycloalkyl, -R¹¹OR¹², -R¹¹SR¹², -C(O)R^{c}, -C(O)OR^{d}, and -C(O)N(R^{a})(R^{b}), or two R³, together with the atoms of ring C to which they are connected, form a 3-10 membered ring structure;
wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{c} is independently selected from the group consisting of H, halogen, and C₁-C₆ alkyl; R^{d} is each independently selected from the group consisting of H and C₁-C₆ alkyl.

In one embodiment, R³ is each independently selected from the group consisting of H, cyclopropyl, isopropyl, tert-butyl, F, Cl, ethyl, methyl, methylcarbonyl, and methoxymethyl.

The present invention relates to a compound of formula (I-2), or stereoisomers, tautomers, solvates, hydrates, active metabolites, isotopic labels, and pharmaceutically acceptable salts thereof: 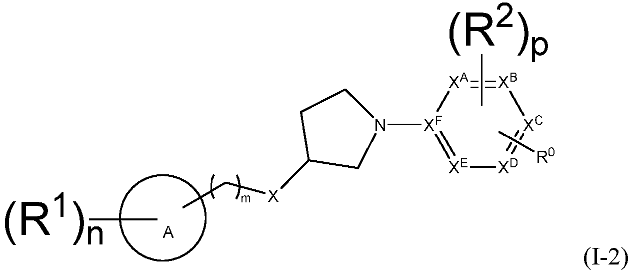
wherein ring A is each independently selected from monocyclic or polycyclic ring systems containing 3-12 ring atoms;
R¹ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}), -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R², together with the atoms of the ring to which they are connected, form a 3-10 membered ring structure;
R⁰ is each independently selected from the group consisting of H, halogen or a structure of formula II 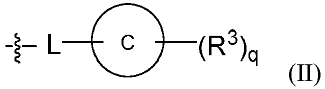
L is each independently selected from the group consisting of a bond, -O-, -S-, -N(R²⁰)-, - C(O)-, -C(R²⁰R²¹)-, -S(O)- and -S(O₂)-;
ring C is each independently selected from monocyclic or polycyclic ring systems containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f};
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f};
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
X is selected from the group consisting of -S-, -S(O)-, and -S(O₂)-;
X^{A}, X^{B}, X^{C}, X^{D} and X^{E} are each independently CH or N;
X^{F} is CH;
n is 0, 1, 2 or 3;
m is 0, 1, 2 or 3;
p is 0, 1, 2 or 3;
q is 0, 1, 2 or 3.
In one embodiment, X^{A}, X^{B}, X^{C}, X^{D}, X^{E} are each independently CH; or
X^{A}, X^{B}, X^{C}, X^{E} are each independently CH, and X^{D} is N; or
X^{A}, X^{B}, X^{C}, X^{D} are each independently CH, and X^{E} is N; or
X^{A}, X^{C}, X^{D} are each independently CH, and X^{B} and X^{E} and are N.

It should be noted that the above restrictions on the functional groups in compound I and compound I-1 also apply to compound I-2.

In the embodiment of the above compounds, m can be 0.

In the embodiment of the above compounds, n can be 1 or 2, that is, ring A is substituted with 1 or 2 R¹.

In the embodiment of the above compounds, p can be 1, that is, the ring B or the hexagonal ring consisting of X^{A}, X^{B}, X^{C}, X^{D}, X^{E}, X^{F} is substituted with one R2. In one embodiment, the R² is located at the ortho position of pyrrolidinyl.

In one embodiment, the compound is selected from the following compounds:

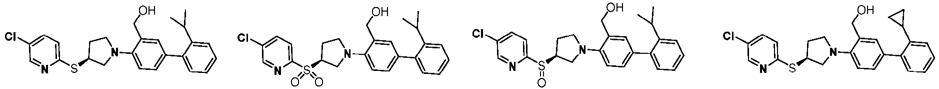

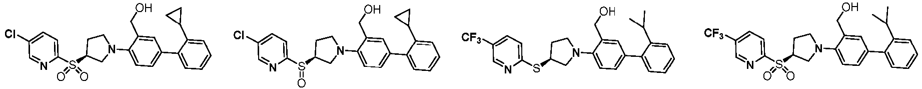

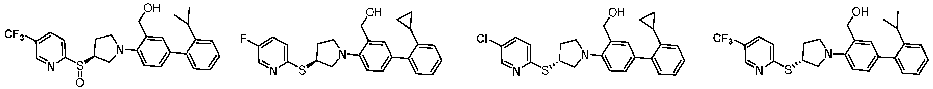

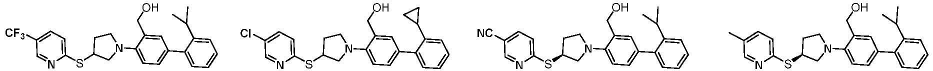

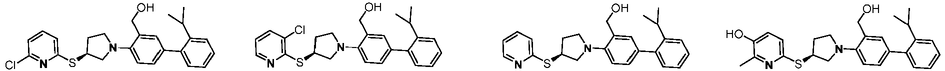

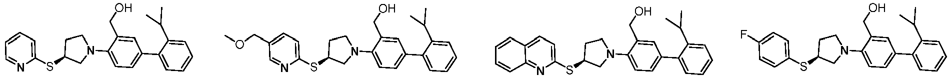

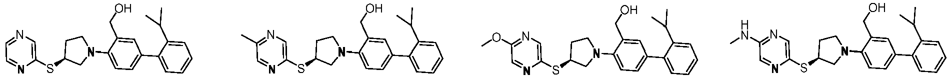

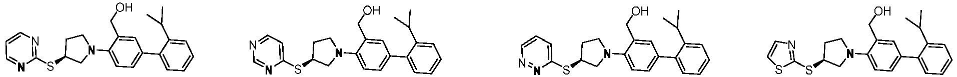

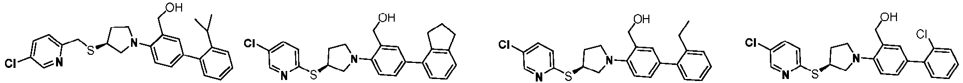

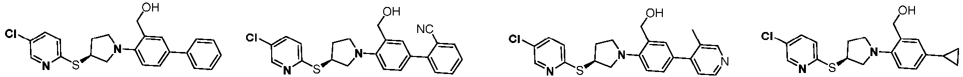

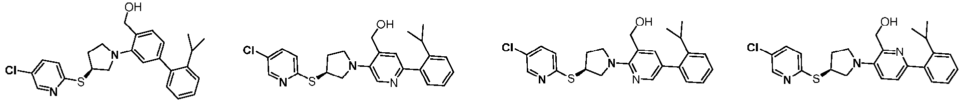

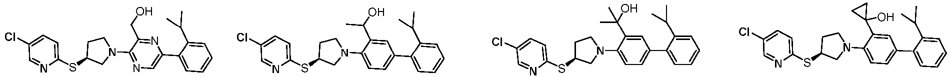

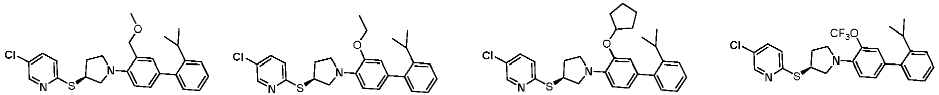

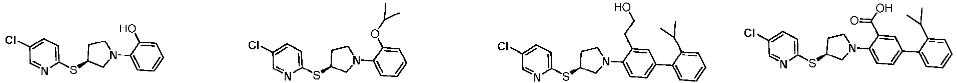

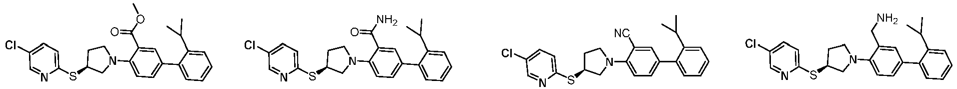

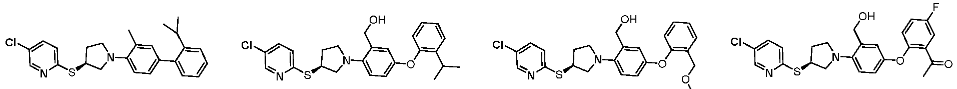

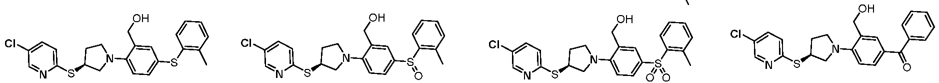

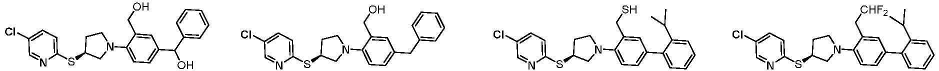

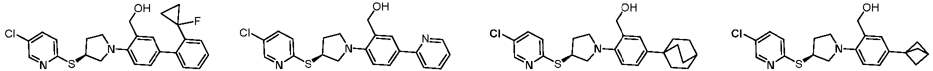

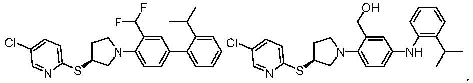

The present invention relates to a pharmaceutical composition, which comprises a compound of the present invention, or stereoisomers, tautomers, solvates, hydrates, active metabolites, isotopic labels, or pharmaceutically acceptable salts thereof, and a pharmaceutically acceptable carrier.

The present invention further relates to use of a compound of the present invention, or stereoisomers, tautomers, solvates, hydrates, active metabolites, isotopic labels, or pharmaceutically acceptable salts thereof, and a pharmaceutical composition of the present invention in manufacture of pharmaceuticals for inhibiting TRPV3 activity.

The present invention further relates to use of a compound of the present invention, or stereoisomers, tautomers, solvates, hydrates, active metabolites, isotopic labels, or pharmaceutically acceptable salts thereof, and a pharmaceutical composition of the present invention in manufacture of pharmaceuticals for treating TRPV3-mediated conditions in a subject.

The present invention further relates to a method for treating TRPV3 mediated conditions, which comprses administering a therapeutically effective amount of a compound of the present invention, or stereoisomers, tautomers, solvates, hydrates, active metabolites, isotopic labels, or pharmaceutically acceptable salts thereof, or a pharmaceutical composition of the present invention to a patient in need of administration.

In one embodiment, the conditions are selected from the group consisting of pain, itching, skin conditions, inflammation, abnormal hair growth, incontinence, fever, hot flashes, cystitis, irritable bowel syndrome, and/or cough symptoms.

In one embodiment, it is used for manufacturing pharmaceuticals that inhibit proliferation, and thereby prevent, treat, or alleviat cancer symptoms.

In one embodiment, the pain is cancerous pain and skin pain.

In one embodiment, the cancer is liposarcoma.

In one embodiment, the abnormal hair growth is hair loss.

In one embodiment, the skin conditions are selected from the group consisting of skin keratosis, ichthyosis, and pruritus.

In one embodiment, the skin keratosis is olmsted syndrome.

In one embodiment, the ichthyosis is harlequin ichtyosis.

### General Synthesis Scheme

The compounds of the present invention can be prepared using the methods illustrated in the general synthesis scheme and experimental procedures detailed below. These general synthesis schemes and experimental procedures are presented for illustrative purposes and are not intended to be limiting. The raw materials used for preparing the compounds of the present invention are commercially available or can be prepared using conventional methods known in the art.

The representative procedures for preparing the compounds of the present invention are summarized in Scheme 1, Scheme 2, and Scheme 3. The raw material (R)-1-BOC-3-hydroxypyrrolidine can be commercially available or prepared using methods known in the art, with representative procedures providing intermediates. Scheme 1 highlights the fully detailed synthesis of (2-(3-(pyridin-2-ylthio)pyrrolidin-1-yl)phenyl)methanol. The synthesis of (*R*)-1-BOC-3-methylsulfonyloxypyrrolidine 2 can be achieved by the reaction of (*R*)-1-BOC-3-hydroxypyrrolidine 1 and methylsulfonyl chloride in a solvent such as dichloromethane, and thioether intermediate 3 is synthesized by reacting (*R*)-1-BOC-3-methylsulfonyloxypyrrolidine 2 with the desired aromatic thiol in an alkaline solution to form intermediate 3. Intermediate 3 is deprotected under acidic conditions to form intermediate 4, which reacts with 5-bromo-2-fluorobenzaldehyde in an alkaline solution to form intermediate 5, which is further reduced to form intermediate 6 under reducing agent conditions. Intermediate 6 can react with the desired boric acid under Suzuki conditions to produce coupling and obtain 7.

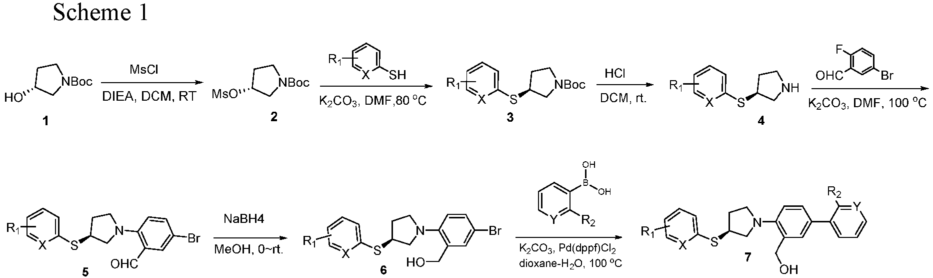

Scheme 2 shows the synthesis of the desired compound, in which 5 is generated through catalytic coupling reaction in the fourth step. The preparation of intermediate 4 is the same as Scheme 1, and intermediate 5 reacts with the desired boric acid under Suzuki conditions to produce coupling and obtain 6.

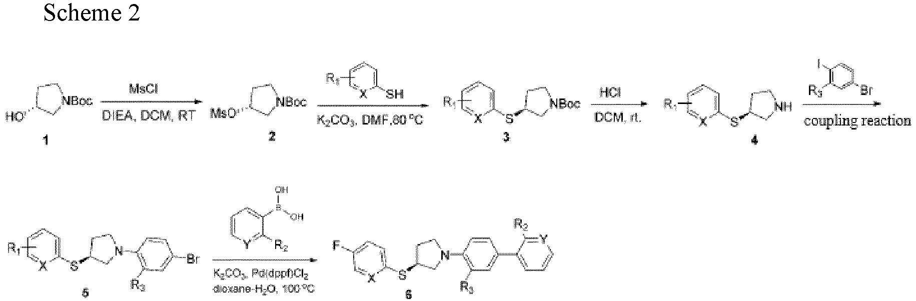

Scheme 3 shows the synthesis of the desired compound, except that intermediate 5 is purchases or prepared by using methods known in the art, firstly. The preparation of intermediate 4 is the same as Scheme 1, and intermediates 4 and 5 generate 6 through catalytic coupling reaction.

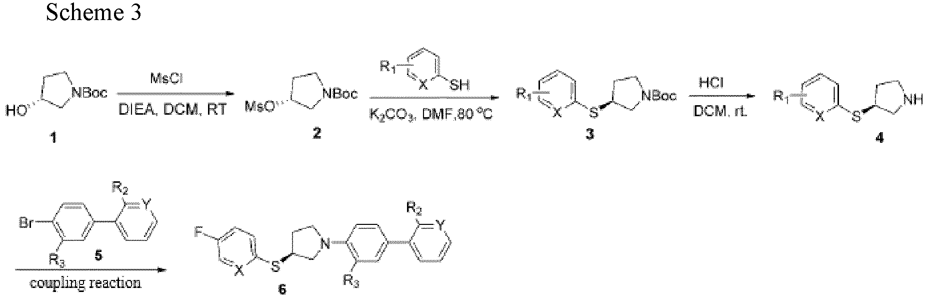

For clarity, further Examples are used to illustrate the present invention, but the Examples do not limit the scope of the present application. All reagents used in the present application are commercially available and can be used without further purification.

### Detailed Description

### Preparation of intermediates

### Preparation Example 1: (S)-5-chloro-2-(pyrrolidin-3-ylthio)pyridine hydrochloride (compound I1)

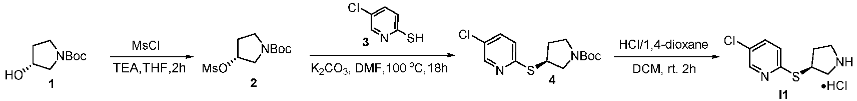

### Step A: (R)-3-(methylsulfonyl)oxy)pyrrolidine-1-tert-butyl carboxylate

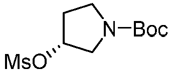

18.0 g (96.1 mmol, 1.0 eq) (*R*)-3-hydroxypyrrolidine-1-tert-butyl carboxylate and 40mL (288 mmol, 3.0 eq) triethylamine were dissolved in 60 mL anhydrous tetrahydrofuran, and cooled to 0 °C, and then 13.2 g (115 mmol, 1.2 eq) methylsulfonyl chloride was added dropwise to the reaction system. After dropwise addition, the reaction system was naturally heated to room temperature and stirred for 2 hours. After confirming that the raw materials had reacted completely by TLC, 150 mL water and 100 mL dichloromethane were added, and then the organic phase was separated. 150 mL dichloromethane was added to the aqueous phase, and an extraction was conducted once. The organic phases were combined, washed with salt water, dried over anhydrous sodium sulfate and concentrated to dryness. The crude product was subjected to column chromatography (petroleum ether: ethyl acetate=100:1~30:1) to obtain a light yellow oily substance (18.2 g, yield=71%).
LC-MS: (M+23)⁺; m/z=288.06;
¹H NMR (400 MHz, CDCl₃) δ 5.18 - 5.13 (m, 1H), 3.60 - 3.32 (m, 4H), 2.93 (s, 3H), 2.16 - 2.02 (m, 2H), 1.35 (s, 9H).

### Step B: (S)-3-(5-chloropyridin-2-yl)thio)pyrrolidine-1-tert-butyl carboxylate

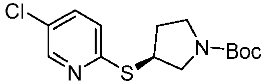

4.00 g (15.1 mmol, 1.0 eq) (*R*)-3-(methylsulfonyl)oxy)pyrrolidine-1-tert-butyl carboxylate was dissolved in 90 mL anhydrous *N, N*-dimethylformamide, and then 3.30 g (22.6 mmol, 1.5 eq) 5-chloropyridine-2-thiol and 3.13 g (22.6 mmol, 1.5eq) potassium carbonate were added to the reaction system. The reaction was carried out under nitrogen protection at 70 °C with stirring for 18 h. After cooling down to room temperature, 50 mL water and 150 mL ethyl acetate were added, and then the organic phase was separated. The aqueous phase was extracted twice with ethyl acetate (2 * 100mL). The organic phases were combined, washed with salt water, dried over anhydrous sodium sulfate and concentrated to obtain a crude product. The crude product was subjected to column chromatography (petroleum ether: ethyl acetate=100:1~20:1) to obtain an oily substance (4.00 g, yield=84%).
LC-MS: (M-100)⁺; m/z= 215.06;
¹H NMR (400 MHz, CDCl₃) δ 8.38 (s, 1H), 7.47 - 7.44 (m, 1H), 7.10 (d, *J =* 8.0 Hz, 1H), 4.32 - 4.27 (m, 1H), 3.93 - 3.84 (m, 1H), 3.59 - 3.27 (m, 3H), 2.37 - 2.32 (m, 1H), 1.99 - 1.92 (m, 1H), 1.46 (s, 9H).

### Step C: (S)-5-chloro-2-(pyrrolidin-3-ylthio)pyridine hydrochloride

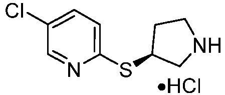

4.30g (13.7mmol, 1.0eq) (*S*)-3-(5-chloropyridin-2-yl)thio)pyrrolidine-1-tert-butyl carboxylate was dissolved in 50 mL dichloromethane, and then 5.7 ml (68.3 mmol, 5.0 eq) concentrated hydrochloric acid was added to the above reaction system. The reaction system was stirred under nitrogen protection at room temperature for 1h. After confirming that the raw materials had reacted completely through LC-MS results, the reaction solution was directly concentrated under reduced pressure to obtain a nearly white solid (2.56 g, yield=88%).
LC-MS:(M+H)⁺; m/z= 415.06

### Intermediate I2: (S)-5-bromo-2-(3-(5-chloropyridin-2-yl)thio)pyrrolidin-1-yl)benzaldehyde

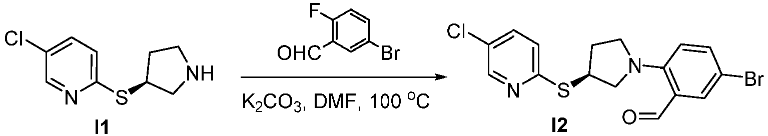

1.50 g (6.99 mmol, 1.0 eq) (*S*)-5-chloro-2-(pyrrolidin-3-ylthio)pyridine was dissolved in 20 mL anhydrous *N*, *N*-dimethylformamide, and then 3.55 g (17.5 mmol, 2.5 eq) 5-bromo-2-fluorobenzaldehyde and 2.90 g (21.0 mmol, 3.0 eq) anhydrous potassium carbonate were added to the above reaction system. The reaction system reacted overnight under nitrogen protection at 90 °C. After confirming that the target product had been generated through LC-MS results, the reaction solution was cooled to room temperature and quenched by adding water. It was extracted twice with ethyl acetate. The organic phases were washed with salt water, dried over anhydrous sodium sulfate and concentrated to dryness, and then subjected to column chromatography (petroleum ether: ethyl acetate=100:1~30:1) to obtain a light yellow oily substance (1.40 g, yield=50%).
LC-MS: (M+H)⁺; m/z= 398.98;
¹H NMR (400 MHz, CDCl₃) δ 10.00 (s, 1H), 8.37 - 8.36 (m, 1H), 7.79 (d, *J =* 4.0 Hz, 1H), 7.48 - 7.44 (m, 2H), 7.10 (d, *J =* 8.0 Hz, 1H), 6.74 (d, *J =* 8.0 Hz, 1H), 4.46 - 4.40 (m, 1H), 3.93 - 3.90 (m, 1H), 3.58 - 3.48 (m, 2H), 3.34 - 3.33 (m, 1H), 2.56 - 2.48 (m, 1H), 2.18 - 2.10 (m, 1H).

### Intermediate I3: (S)-(5-bromo-2-(3-((5-chloropyridin-2-yl)thio)pyrrolidin-1-yl)phenyl) methanol

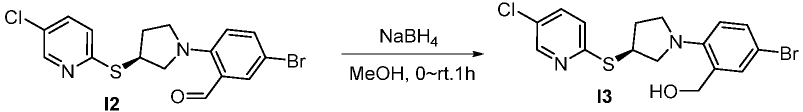

2.0 g (5.03 mmol, 1.0 eq) (S)-5-bromo-2-(3-(5-chloropyridin-2-yl)thio)pyrrolidin-1-yl) benzaldehyde was dissolved in 10 mL methanol, and the system was cooled to 0 °C. 761 mg (20.12 mmol, 4.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. Distillation and concentration under reduced pressure were carried out to obtain the target product (1.73 g, yield=86%).

LC-MS: (M+H)⁺; m/z= 400.98.

### Example 1: ((S)-(4-(3-(5-chloropyridin-2-yl)thio)pyrrolidin-1-yl)-2'-isopropyl-[1,1'-biphenyl]-3-yl)methanol

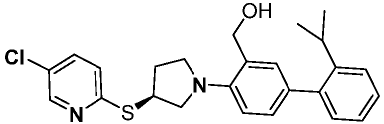

### Step A: (S)-4-(3-(5-chloropyridin-2-yl)thio)pyrrolidin-1-yl)-2'-isopropyl-[1,1'-biphenyl]-3-formaldehyde

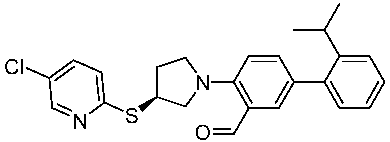

900 mg (2.26 mmol, 1.0 eq) (*S*)-5-bromo-2-(3-(5-chloropyridin-2-yl)thio)pyrrolidin-1-yl) benzaldehyde was dissolved in 20 mL dioxane and 5 mL water, and then 445 mg (2.72 mmol, 1.2 eq) (2-isopropylphenyl)boric acid and 626 mg (4.53 mmol, 2.0 eq) anhydrous potassium carbonate were added to the above reaction system. 185 mg (0.23 mmol, 0.1 eq) [1,1'-bis(diphenylphosphine)ferrocene] dichloride palladium was added under nitrogen protection and reacted at 100 °C for 4 hours. After confirming that the target product had been generated through LC-MS results, the reaction solution was cooled to room temperature and quenched by adding water. It was extracted twice with ethyl acetate. The organic phases were washed with salt water, dried over anhydrous sodium sulfate and concentrated to dryness, and then subjected to column chromatography (petroleum ether: ethyl acetate=100:1~30:1) to obtain a light yellow oily substance (710 mg, yield=71%).
LC-MS: (M+H)⁺; m/z= 437.16;
¹H NMR (400 MHz, CDCl₃) δ 10.13 (s, 1H), 8.41 - 8.40 (m, 1H), 7.66 (d, *J =* 4.0 Hz, 1H), 7.50 (dd, *J =* 8.0 Hz, 4.0 Hz, 1H),7.42 - 7.34 (m, 3H), 7.25 - 7.14 (m, 3H), 6.93 (d, *J =* 8.0 Hz, 1H), 4.53 - 4.46 (m, 1H), 4.01 - 3.99 (m, 1H), 3.67 - 3.60 (m, 2H), 3.46 - 3.45 (m, 1H), 3.13 - 3.04 (m, 1H), 2.63 - 2.55 (m, 1H), 2.26 - 2.15 (m, 1H), 1.20 (d, *J =* 8.0 Hz, 6H).

### Step B: (S)-4-(3-(5-chloropyridin-2-yl)thio)pyrrolidin-1-yl)-2'-isopropyl-[1,1'-biphenyl]-3-methanol

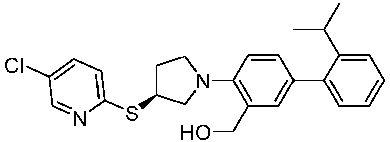

150 mg (0.34 mmol, 1.0 eq) ((*S*)-4-(3-(5-chloropyridin-2-yl)thio)pyrrolidin-1-yl)-2'-isopropyl-[1,1'-biphenyl]-3-formaldehyde was dissolved in 10 mL methanol, and the system was cooled to 0 °C. 51.9 mg (1.37 mmol, 4.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product (80.0 mg, yield=53%).
LC-MS:(M+H)⁺; m/z= 439.18;
¹H NMR (400 MHz, CDCl₃) δ 8.39 (d, *J =* 4.0 Hz, 1H), 7.46 (dd, *J =* 8.0 Hz, 4.0 Hz, 1H), 7.38 - 7.30 (m, 2H),7.21 - 7.12 (m, 6H), 4.82 - 4.78 (m, 2H), 4.46 - 4.40 (m, 1H), 4.20 (s, 1H), 3.76 - 3.75 (m, 1H), 3.49 - 3.44 (m, 1H) , 3.32 - 3.25 (m, 2H), 3.09 - 3.03 (m, 1H), 2.63 - 2.54 (m, 1H), 2.11- 2.03 (m, 1H), 1.15 (d, *J =* 4.0 Hz, 6H).

### Example 2: ((S)-(4-(3-(5-chloropyridin-2-yl)sulfonyl)pyrrolidin-1-yl)-2'-isopropyl-[1,1'-biphenyl]-3-yl)methanol

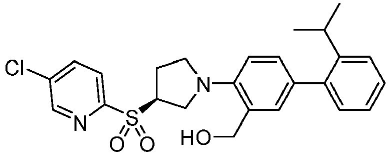

### Step A: (S)-4-(3-(5-chloropyridin-2-yl)sulfonyl)pyrrolidin-1-yl)-2'-isopropyl-[1,1'-biphenyl]-3-formaldehyde

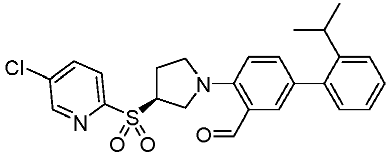

240 mg (0.55 mmol, 1.0 eq) (*S*)-4-(3-(5-chloropyridin-2-yl)thio)pyrrolidin-1-yl)-2'-isopropyl-[1,1'-biphenyl]-3-formaldehyde was dissolved in 6 mL methanol and 3 mL tetrahydrofuran, and then 124 mg (0.55 mmol, 1.0 eq) sodium tungstate and 0.5 mL (5.5 mmol, 10.0 eq) hydrogen peroxide solution were added to the reaction system. It was heated to 40 °C and reacted for 2 h. After confirming that the target product had been generated through LC-MS results, the reaction solution was cooled to room temperature and quenched by adding water. It was extracted twice with ethyl acetate. The organic phases were washed with salt water, dried over anhydrous sodium sulfate and concentrated to dryness, and then subjected to column chromatography (petroleum ether: ethyl acetate=100:1~3:1) to obtain a light yellow oily substance (180 mg, yield=69%).
LC-MS: (M+H)⁺; m/z= 471.17;

### Step B: (S)-(4-(3-(5-chloropyridin-2-yl)sulfonyl)pyrrolidin-1-yl)-2'-isopropyl-[1,1'-biphenyl]-3-yl)methanol

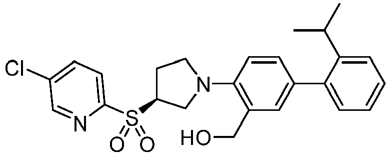

180 mg (0.38 mmol, 1.0 eq) (5)-4-(3-(5-chloropyridin-2-yl)sulfonyl)pyrrolidin-1-yl)-2'-isopropyl-[1,1'-biphenyl]-3-formaldehyde was dissolved in 5 mL methanol, and the system was cooled to 0 °C. 72.6 mg (1.37 mmol, 5.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product (81.8 mg, yield=45%).
LC-MS: (M+H)⁺; m/z= 471.18;
¹H NMR (400 MHz, CDCl₃) δ 8.70 (d, *J =* 2.0 Hz, 1H), 8.12 (d, *J =* 8.0 Hz, 1H), 7.96 (dd, *J* = 8.0 Hz, 4.0 Hz, 1H), 7.38 - 7.30 (m, 2H), 7.20 - 7.11 (m, 5H), 4.83 (d, *J =* 12.0 Hz, 1H), 4.70 (d, *J =* 16.0 Hz, 1H), 4.50 - 4.40 (m, 1H) , 4.0 (s, 1H), 3.68 - 3.66 (m, 1H), 3.49 - 3.43 (m, 2H), 3.24 - 3.18 (m, 1H), 3.07 - 3.00 (m, 1H), 2.64 - 2.55 (m, 1H), 2.42 - 2.33 (m, 1H), 1.15 (d, *J =* 6.8 Hz, 6H).

### Example 3: (4-(S)-3-(S)-(5-chloropyridin-2-yl)sulfoxido)pyrrolidin-1-yl)-2'-isopropyl-[1,1'-biphenyl]-3-yl)methanol

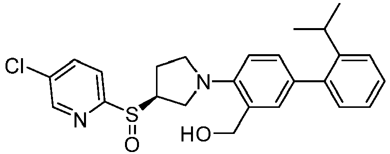

### Step A: 4-(S)-3-(S)-(5-chloropyridin-2-yl)sulfoxido)pyrrolidin-1-yl)-2'-isopropyl-[1,1'-biphenyl]-3-formaldehyde

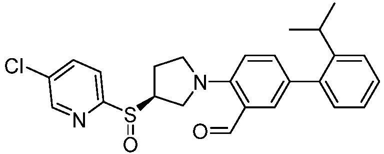

230 mg (0.53 mmol, 1.0 eq) (*S*)-4-(3-(5-chloropyridin-2-yl)thio)pyrrolidin-1-yl)-2'-isopropyl-[1,1'-biphenyl]-3-carbaldehyde was dissolved in 5 mL dichloromethane. The system was cooled to 0 °C under nitrogen protection, and then 145 mg (0.84 mmol, 1.6 eq) m-chloroperoxybenzoic acid was added to the reaction system, and reacted at 0 °C for 2 h. After confirming that the target product had been generated through LC-MS results, the reaction solution was quenched by adding water. It was extracted twice with dichloromethane. The organic phases were washed with salt water, dried over anhydrous sodium sulfate and concentrated to dryness, and then subjected to column chromatography (petroleum ether: ethyl acetate=80:1~1:1) to obtain a light yellow oily substance (180 mg, yield=75%).
LC-MS: (M+H)⁺; m/z = 453.17;

### Step B: (4-(S)-3-(S)-(5-chloropyridin-2-yl)sulfoxido)pyrrolidin-1-yl)-2'-isopropyl-[1,1'-biphenyl]-3-yl)methanol

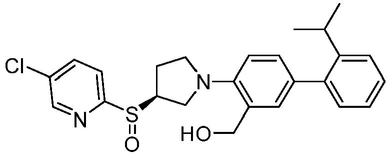

170 mg (0.38 mmol, 1.0 eq) (4-(*S*)-3-(*S*)-(5-chloropyridin-2-yl)sulfoxido)pyrrolidin-1-yl)-2'-isopropyl-[1,1'-biphenyl]-3-carbaldehyde was dissolved in 5 mL methanol, and the system was cooled to 0 °C. 42.6 mg (1.13 mmol, 3.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product as white solid (68.5 mg, yield=40%).
LC-MS: (M+H)⁺; m/z= 455.16;
¹H NMR (400 MHz, CDCl₃) δ 8.61 - 8.59 (m, 1H), 8.00 (dd, *J =* 8.0 Hz, 4.0 Hz, 1H), 7.95 - 7.90 (m, 1H), 7.39 - 7.30 (m, 2H), 7.21 - 7.10 (m, 5H), 4.86 - 4.80 (m, 1H), 4.71 - 4.69 (m, 1H), 3.85 - 3.78 (m, 2H), 3.66 - 3.52 (m, 1H), 3.44 - 3.35 (m, 1H), 3.14 - 3.00 (m, 2H), 2.66 - 2.59 (m, 1H), 2.56 - 2.39 (m, 1H), 1.94 - 1.85 (m, 1H), 1.16 (d, *J =* 6.8 Hz, 6H).

### Example 4: ((S)-(4-(3-(5-chloropyridin-2-yl)thio)pyrrolidin-1-yl)-2'-cyclopropyl-[1,1'-biphenyl]-3-yl)methanol

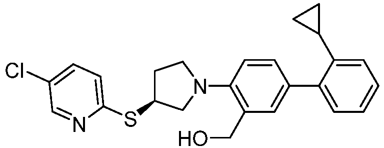

### Step A: (S)-4-(3-(5-chloropyridin-2-yl)thio)pyrrolidin-1-yl)-2'-cyclopropyl-[1,1'-biphenyl]-3-formaldehyde

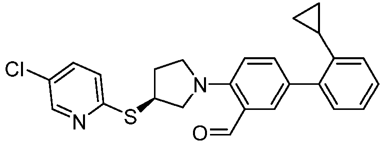

600 mg (1.50 mmol, 1.0eq) (*S*)-5-bromo-2-(3-(5-chloropyridin-2-yl)thio)pyrrolidin-1-yl)benzaldehyde was dissolved in 20 mL dioxane and 5 mL water, and then 293 mg (1.81 mmol, 1.2 eq) (2-cyclopropylphenyl)boric acid and 626 mg (4.53 mmol, 3.0 eq) anhydrous potassium carbonate were added to the above reaction system. 221 mg (0.23 mmol, 0.2 eq) [1,1'-bis(diphenylphosphine)ferrocene] dichloride palladium was added under nitrogen protection and reacted at 100 °C for 4 hours. After confirming that the target product had been generated through LC-MS results, the reaction solution was cooled to room temperature and quenched by adding water. It was extracted three times with ethyl acetate. The organic phases were washed with salt water, dried over anhydrous sodium sulfate and concentrated to dryness, and then subjected to column chromatography (petroleum ether: ethyl acetate=100:1~30:1) to obtain a light yellow oily substance (450 mg, yield=68%).
LC-MS: (M+H)⁺; m/z= 435.12;

### Step B: ((S)-(4-(3-(5-chloropyridin-2-yl)thio)pyrrolidin-1-yl)-2'-cyclopropyl-[1,1'-biphenyl]-3-yl)methanol

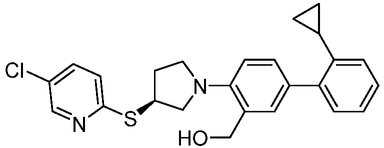

480 mg (1.10 mmol, 1.0 eq) ((*S*)-4-(3-(5-chloropyridin-2-yl)thio)pyrrolidin-1-yl)-2'-cyclopropyl-[1,1'-biphenyl]-3-carbaldehyde was dissolved in 10 mL methanol, and the system was cooled to 0 °C. 209 mg (5.52 mmol, 4.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product as a light yellow oily liquid (360 mg, yield=74%).
LC-MS: (M+H)⁺; m/z= 437.30;
¹H NMR (400 MHz, CDCl₃) δ 8.41 (d, *J =* 4.0 Hz, 1H), 7.46 (dd, *J =* 8.0 Hz, 4.0 Hz, 1H), 7.37 - 7.33 (m, 2H),7.28 - 7.25 (m, 1H), 7.23 - 7.20 (m, 2H), 7.18 - 7.15 (m, 2H), 6.93 (d, *J=* 8.0 Hz, 1H), 4.86 - 4.80 (m, 2H), 4.49 - 4.44 (m, 1H), 4.21 (s, 1H), 3.79 - 3.78 (m, 1H), 3.52 - 3.46 (m, 1H) , 3.35 - 3.27 (m, 2H), 2.65 - 2.57 (m, 1H), 2.13 - 2.05 (m, 1H), 1.95 - 1.88 (m, 1H), 0.89 - 0.84 (m, 2H), 0.75 - 0.71 (m, 2H).

### Example 5 : (S)-(4-(3-((5-chloropyridin-2-yl)sulfonyl)pyrrolidin-1-yl)-2'-cyclopropyl-[1,1'-biphenyl]-3-yl)methanol

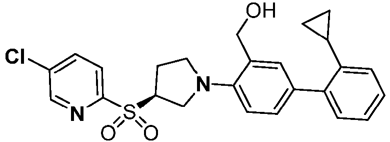

The experimental operation follows the synthesis steps of Example 2, except that the reagent (2-isopropylphenyl) boronic acid was replaced with (2-cyclopropylphenyl) boronic acid, and synthesized to obtain Example 5.
LC-MS: (M+H)⁺; m/z= 469.11;
¹H NMR (400 MHz, CDCl₃) δ 8.71 (d, *J =* 2.0 Hz, 1H), 8.13 (d, *J =* 8.0 Hz, 1H), 7.98 (dd, *J =* 8.0 Hz, 4.0 Hz, 1H), 7.39 - 7.31 (m, 2H), 7.22 - 7.11 (m, 5H), 4.85 - 4.83 (m, 1H), 4.71 - 4.69 (m, 1H), 4.50 - 4.40 (m, 1H) , 4.0 (s, 1H), 3.68 - 3.66 (m, 1H), 3.49 - 3.43 (m, 2H), 3.24 - 3.18 (m, 1H), 2.66 - 2.57 (m, 1H), 2.14 - 2.06 (m, 1H), 1.95 - 1.88 (m, 1H), 0.89 - 0.85 (m, 2H), 0.75 - 0.72 (m, 2H).

### Example 6: (4-(S)-3-((S)-(5-chloropyridin-2-yl)sulfoxido)pyrrolidin-1-yl)-2'-cyclopropyl-[1,1'-biphenyl]-3-yl)methanol

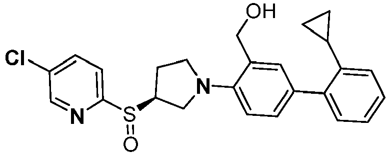

The experimental operation follows the synthesis steps of Example 3, except that the reagent (2-isopropylphenyl) boronic acid was replaced with (2-cyclopropylphenyl) boronic acid, and synthesized to obtain Example 6.
LC-MS: (M+H)⁺; m/z= 453.10;
¹H NMR (400 MHz, CDCl₃) δ 8.63 - 8.59 (m, 1H), 8.01 (dd, *J =* 8.0 Hz, 4.0 Hz, 1H), 7.95 - 7.90 (m, 1H), 7.38 - 7.29 (m, 2H), 7.21 - 7.10 (m, 5H), 4.86 - 4.80 (m, 1H), 4.71 - 4.69 (m, 1H), 3.85 - 3.78 (m, 2H) , 3.66 - 3.52 (m, 1H), 3.44 - 3.35 (m, 1H), 3.26 - 3.20 (m, 1H), 3.14 - 3.00 (m, 1H), 2.65 - 2.57 (m, 1H), 2.13 - 2.05 (m, 1H), 1.95 - 1.88 (m, 1H), 0.88 - 0.84 (m, 2H), 0.76 - 0.71 (m, 2H).

### Example 7: ((S)-(2'-isopropyl-4-(3-(5-(trifluoromethyl)pyridin-2-yl)thio)pyrrolidin-1-yl)-[1,1'-biphenyl]-3-yl)methanol

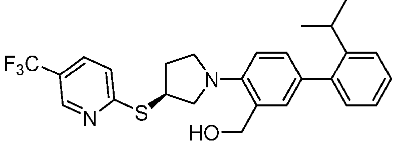

### Step A: (S)-3-(5-(trifluoromethyl)pyridin-2-yl)thio)pyrrolidine-1-tert-butyl carboxylate

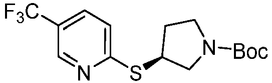

5.00 g (18.9 mmol, 1.0 eq) (*R*)-3-(methylsulfonyl)oxy)pyrrolidine-1-tert-butyl carboxylate was dissolved in 90 mL anhydrous *N*, *N*-dimethylformamide, and then 5.06 g (28.3 mmol, 1.5 eq) 5-(trifluoromethyl)pyridine-2-thiol and 3.91 g (28.3 mmol, 1.5 eq) potassium carbonate were added to the reaction system. The reaction was carried out under nitrogen protection at 70 °C with stirring for 18 h. After cooling down to room temperature, 50 mL water and 150 mL ethyl acetate were added, and then the organic phase was separated. The aqueous phase was extracted three times with ethyl acetate (2 * 100mL). The organic phases were combined, washed with salt water, dried over anhydrous sodium sulfate and concentrated to obtain a crude product. The crude product was subjected to column chromatography (petroleum ether: ethyl acetate=100:1~10:1) to obtain an oily substance (5.80 g, yield=87%).
LC-MS: (M-56)⁺; m/z= 293.03;

### Step B: (S)-2-(pyrrolidin-3-ylthio)-5-(trifluoromethyl)pyridine hydrochloride

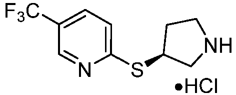

4.20 g (12.1 mmol, 1.0 eq) (*S*)-3-(5-(trifluoromethyl)pyridin-2-yl)thio)pyrrolidine-1-tert-butyl carboxylate was dissolved in 50 mL dichloromethane, and then 5.7 ml (68.3 mmol, 5.0 eq) concentrated hydrochloric acid was added to the above reaction system. The reaction system was stirred under nitrogen protection at room temperature for 1h. After confirming that the raw materials had reacted completely through LC-MS results, the reaction solution was directly concentrated under reduced pressure to obtain a nearly white solid (3.10 g, yield=99%).
LC-MS: (M+H)⁺; m/z= 249.09;

### Step C: (S)-5-bromo-2-(3-(5-(trifluoromethyl)pyridin-2-yl)thio)pyrrolidin-1-yl)benzaldehyde

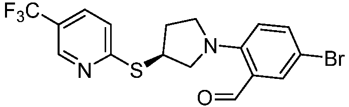

2.50 g (10.1 mmol, 1.0 eq.) (S)-2-(pyrrolidin-3-ylthio)-5-(trifluoromethyl)pyridine hydrochloride was dissolved in 30 mL anhydrous *N*, *N*-dimethylformamide, and then 5.11 g (25.2 mmol, 2.5 eq) 5-bromo-2-fluorobenzaldehyde and 3.20 g (30.2 mmol, 3.0 eq) anhydrous sodium carbonate were added to the above reaction system. The reaction system reacted overnight under nitrogen protection at 100 °C. After confirming that the target product had been generated through LC-MS results, the reaction solution was cooled to room temperature and quenched by adding water. It was extracted three times with ethyl acetate. The organic phases were washed with salt water, dried over anhydrous sodium sulfate and concentrated to dryness, and then subjected to column chromatography (petroleum ether: ethyl acetate=100:1~20:1) to obtain a light yellow oily substance (3.14 g, yield=72%).
LC-MS: (M+H)⁺; m/z= 432.94;
¹H NMR (400 MHz, CDCl₃) δ 10.00 (s, 1H), 8.65 - 8.64 (m, 1H), 7.80 (d, *J =* 4.0 Hz, 1H), 7.67 (dd, *J =* 8.0 Hz, 4.0 Hz, 1H), 7.46 (dd, *J =* 8.0 Hz, 4.0 Hz, 1H), 7.26 - 7.24 (m, 1H), 6.76 (d, *J =* 12.0 Hz, 1H), 4.56 - 4.50 (m, 1H), 3.94 - 3.93 (m, 1H), 3.60 - 3.49 (m, 2H), 3.36 - 3.35 (m, 1H), 2.60 - 2.52 (m, 1H), 2.22 - 2.12 (m, 1H).
¹⁹F NMR (376 MHz, CDCl₃) δ -62.21.

### Step D: (S)-2'-isopropyl-4-(3-(5-(trifluoromethyl)pyridin-2-yl)thio)pyrrolidin-1-yl)-[1,1'-biphenyl]-3-formaldehyde

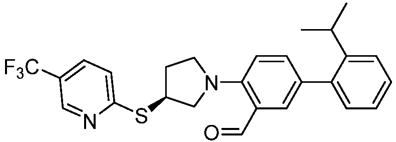

1.55 g (3.60 mmol, 1.0 eq) (*S*)-5-bromo-2-(3-(5-(trifluoromethyl)pyridin-2-yl)thio)pyrrolidin-1-yl)benzaldehyde was dissolved in 30 mL1,4-dioxane and 6 mL water, and then 707 mg (4.31 mmol, 1.2 eq) (2-isopropylphenyl)boric acid and 993 mg (7.19 mmol, 2.0 eq) anhydrous potassium carbonate were added to the above reaction system. 263 mg (0.36 mmol, 0.1 eq) [1,1'-bis(diphenylphosphine)ferrocene] dichloride palladium was added under nitrogen protection and reacted at 100 °C for 3 hours. After confirming that the target product had been generated through LC-MS results, the reaction solution was cooled to room temperature and quenched by adding water. It was extracted twice with ethyl acetate. The organic phases were washed with salt water, dried over anhydrous sodium sulfate and concentrated to dryness, and then subjected to column chromatography (petroleum ether: ethyl acetate=100:1~20:1) to obtain a light yellow oily substance (1.30 g, yield=77%).
LC-MS: (M+H)⁺; m/z= 471.20;

### Step E: ((S)-(2'-isopropyl-4-(3-(5-(trifluoromethyl)pyridin-2-yl)thio)pyrrolidin-1-yl)-[1,1'-biphenyl]-3-yl)methanol

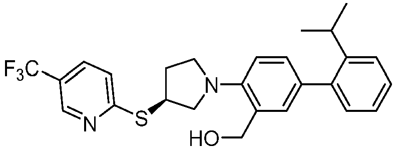

300 mg (0.64 mmol, 1.0 eq) ((*S*)-2'-isopropyl-4-(3-(5-(trifluoromethyl)pyridin-2-yl)thio)pyrrolidin-1-yl)-[1,1'-biphenyl]-3-formaldehyde was dissolved in 6 mL methanol, and the system was cooled to 0 °C. 121 mg (3.19 mmol, 5.0 eq) sodium borohydride solid was added to the above reaction system under nitrogen protection. After the addition, the reaction system was naturally heated to room temperature and reacted for 1h. After confirming that the target product had been generated through LC-MS results, the reaction solution was cooled to 0 °C and quenched by adding saturated ammonium chloride aqueous solution. It was extracted three times with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product as a light yellow oily liquid (75.2 mg, yield=25%).
LC-MS: (M+H)⁺; m/z= 473.23 ;
¹H NMR (400 MHz, CDCl₃) δ 8.67 (s, 1H), 7.68 (d, *J =* 8.0 Hz, 1H), 7.38 - 7.27 (m, 3H), 7.21 - 7.13 (m, 5H), 4.82 - 4.75 (m, 2H), 4.56 - 4.50 (m, 1H), 4.14 (s, 1H), 3.82 - 3.78 (m, 1H) , 3.51 - 3.45 (m, 1H), 3.35 - 3.26 (m, 2H), 3.10 - 3.03 (m, 1H), 2.66- 2.58 (m, 1H), 2.14- 2.05 (m, 1H), 1.15 (d, *J =* 4.0 Hz, 6H).
¹⁹F NMR (376 MHz, CDCl₃) δ -62.17.

### Example 8: (S)-(2'-isopropyl-4-(3-(5-(trifluoromethyl)pyridin-2-yl)sulfonyl)pyrrolidin-1-yl)-[1,1'-biphenyl]-3-yl)methanol

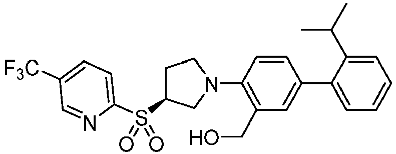

### Step A: (S)-2'-isopropyl-4-(3-(5-(trifluoromethyl)pyridin-2-yl)sulfonyl)pyrrolidin-1-yl)-[1,1'-biphenyl]-3-formaldehyde

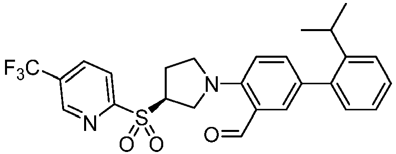

200 mg (0.43 mmol, 1.0 eq) (*S*)-2'-isopropyl-4-(3-(5-(trifluoromethyl)pyridin-2-yl)thio)pyrrolidin-1-yl)-[1,1'-biphenyl]-3-formaldehyde was dissolved in 4 mL methanol and 2 mL tetrahydrofuran, and then 96.0 mg (0.43 mmol, 1.0 eq) sodium tungstate and 0.5 mL (4.3 mmol, 10.0 eq) hydrogen peroxide solution were added to the reaction system. It was heated to 40 °C and reacted for 2 h. After confirming that the target product had been generated through LC-MS results, the reaction solution was cooled to room temperature and quenched by adding water. It was extracted three times with ethyl acetate. The organic phases were washed with salt water, dried over anhydrous sodium sulfate and concentrated to dryness, and then subjected to column chromatography (petroleum ether: ethyl acetate=100:1~10:1) to obtain a light yellow oily substance (115 mg, yield=54%).
LC-MS: (M+H)⁺; m/z= 503.17;

### Step B: (S)-(2'-isopropyl-4-(3-(5-(trifluoromethyl)pyridin-2-yl)sulfonyl)pyrrolidin-1-yl)-[1,1'-biphenyl]-3-yl)methanol

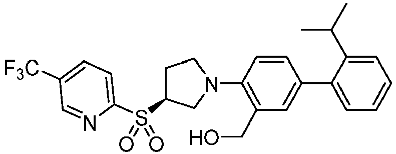

115 mg (0.23 mmol, 1.0 eq) (*S*)-2'-isopropyl-4-(3-(5-(trifluoromethyl)pyridin-2-yl)sulfonyl)pyrrolidin-1-yl)-[1,1'-biphenyl]-3-formaldehyde was dissolved in 5 mL methanol, and the system was cooled to 0 °C. 43.3 mg (1.14 mmol, 5.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product as white solid (42.6 mg, yield=37%).
LC-MS: (M+H)⁺; m/z= 505.22;
¹H NMR (400 MHz, CDCl₃) δ 9.02 - 9.01 (m, 1H), 8.32 (d, *J =* 8.0 Hz, 1H), 8.26 - 8.24 (m, 1H), 7.38 - 7.31 (m, 2H), 7.21 - 7.11 (m, 5H), 4.83 (d, *J =* 12.0 Hz, 1H), 4.71 - 4.69 (m, 1H), 4.55 - 4.48 (m, 1H) , 3.93 (s, 1H), 3.68 - 3.67 (m, 1H), 3.52 - 3.45 (m, 2H), 3.25 - 3.20 (m, 1H), 3.06 - 3.00 (m, 1H), 2.66 - 2.58 (m, 1H), 2.46 - 2.37 (m, 1H), 1.15 (d, *J =* 7.2 Hz, 6H).
¹⁹F NMR (376 MHz, CDCl₃) δ -62.65.

### Example 9 : (2' -isopropyl-4-(S)-3-(S)-(5-(trifluoromethyl)pyridin-2-yl)sulfoxido)pyrrolidin-1-yl)-[1,1'-biphenyl]-3-yl)methanol

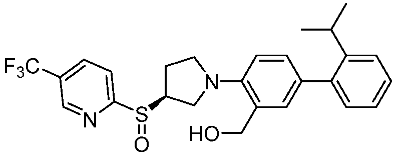

### Step A: 2'-isopropyl-4-(S)-3-(S)-(5-(trifluoromethyl)pyridin-2-yl)sulfoxido)pyrrolidin-1-yl)-[1,1'-biphenyl]-3-formaldehyde

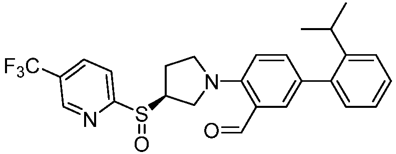

390 mg (0.83 mmol, 1.0 eq) ((*S*)-2'-isopropyl-4-(3-(5-(trifluoromethyl)pyridin-2-yl)thio)pyrrolidin-1-yl)-[1,1'-biphenyl]-3-formaldehyde was dissolved in 5 mL dichloromethane. The system was cooled to 0 °C under nitrogen protection, and then 215 mg (1.24 mmol, 1.5 eq) m-chloroperoxybenzoic acid was added to the reaction system, and reacted at 0 °C for 2 h. After confirming that the target product had been generated through LC-MS results, the reaction solution was quenched by adding water. It was extracted three times with dichloromethane. The organic phases were washed with salt water, dried over anhydrous sodium sulfate and concentrated to dryness, and then subjected to column chromatography (petroleum ether: ethyl acetate=50:1~1:1) to obtain a light yellow oily substance (300 mg, yield=74%).
LC-MS: (M+H)⁺; m/z= 487.37;

### Step B: (2'-isopropyl-4-(S)-3-(S)-(5-(trifluoromethyl)pyridin-2-yl)sulfoxido)pyrrolidin-1-yl)-[1,1'-biphenyl]-3-yl)methanol

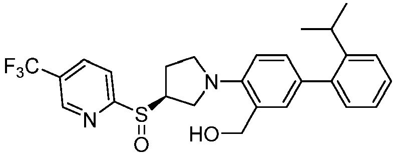

300 mg (0.62 mmol, 1.0 eq) 2'-isopropyl-4-(*S*)-3-(*S*)-(5-(trifluoromethyl)pyridin-2-yl)sulfoxido)pyrrolidin-1-yl)-[1,1'-biphenyl]-3-formaldehyde was dissolved in 5 mL methanol, and the system was cooled to 0 °C. 70.0 mg (1.85 mmol, 3.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted twice with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain the target product as white solid (92.3 mg, yield=31%).
LC-MS: (M+H)⁺; m/z= 489.20;
¹H NMR (400 MHz, CDCl₃) δ 8.93 (d, *J =* 8.0 Hz, 1H), 8.23 - 8.21 (m, 2H), 7.41 - 7.32 (m, 2H), 7.24 - 7.13 (m, 5H), 4.87 - 4.85 (m, 1H), 4.78 - 4.67 (m, 1H), 3.94 - 3.86 (m, 2H), 3.72 - 3.67 (m, 0.5H), 3.58 - 3.54 (m, 0.5H), 3.48 - 3.39 (m, 1H), 3.30 - 3.24 (m, 0.5H), 3.17 - 3.02 (m, 2H), 2.74 - 2.67 (m, 0.5H), 2.63 - 2.42 (m, 1H), 1.96 - 1.87 (m, 1H), 1.18 (d, *J =* 6.8 Hz, 6H).
¹⁹F NMR (376 MHz, CDCl₃) δ -62.32 - -62.35.

### Example 10: ((S)-(4-(3-(5-fluoropyridin-2-yl)thio)pyrrolidin-1-yl)-2'-cyclopropyl-[1,1'-biphenyl]-3-yl)methanol

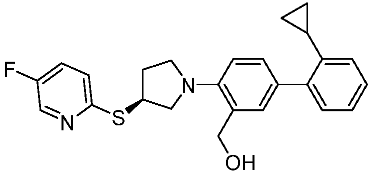

### Step A: 5-fluoropyridine-2-thiol

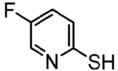

50 mL (125.00 mmol, 1.1 eq) n-butyl lithium was dissolved in 300mL toluene, cooled down to -78 °C, and 20 g (113.64 mmol, 1.0 eq) 2-bromo-5-fluoropyridine was dissolved in 50mL toluene, and then added dropwise to a reaction flask. The reaction was carried out under insulation for 1 h. 3.64 g (114 mmol, 1.0 eq) sulfur powder was weighed, and added in batches to the reaction solution, and the temperature was maintianed at -78 °C for 30 minutes. It was naturally heated to room temperature and reacted for 1 h. A complete reaction was confirmed through TLC Plate (petroleum ether: ethyl acetate=1:1). 20 mL water was added for quenching. The pH was adjusted to 3~4 by using 1N dilute hydrochloric acid solution. Dichloromethane (200 mL) was used to extract five times. The organic phases were combined, washed with salt water, dried over anhydrous sodium sulfate and concentrated to obtain a crude product. The crude product was triturated with ethyl acetate and filtered to obtain a pure yellow solid (6.5 g, yield=44%).
¹H NMR (400 MHz, CDCl₃) δ 7.68 (d, *J =* 4.0 Hz, 1H), 7.50 (d, *J =* 4.0 Hz, 1H), 7.29 - 7.34 (m, 1H).

### Step B: (S)-3-(5-fluoropyridin-2-yl)thio)pyrrolidine-1-tert-butyl carboxylate

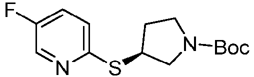

1.00 g (4.01 mmol, 1.0 eq) (*R*)-3-(methylsulfonyl)oxy)pyrrolidine-1-tert-butyl carboxylate was dissolved in 10 mL anhydrous *N*, *N*-dimethylformamide, and then 0.52 g (4.01 mmol, 1.0 eq) 5-fluoropyridine-2-thiol and 1.66 g (12.0 mmol, 3.0 eq) potassium carbonate were added to the reaction system. The reaction was carried out under nitrogen protection at 70 °C with stirring for 18 h. After cooling down to room temperature, 50 mL water and 150 mL ethyl acetate were added, and then the organic phase was separated. The aqueous phase was extracted twice with ethyl acetate (2 * 100mL). The organic phases were combined, washed with salt water, dried over anhydrous sodium sulfate and concentrated to obtain a crude product. The crude product was subjected to column chromatography (petroleum ether: ethyl acetate=10:1) to obtain an oily substance (1.00 g, yield=83%).
LC-MS: (M+H)⁺; m/z= 299.22;
¹H NMR (400 MHz, CDCl₃) δ 8.33 - 7.34 (m, 1H), 7.26 - 7.31 (m, 1H), 7.17 - 7.20 (m, 1H), 4.29 - 4.32 (m, 1H), 3.86 - 3.95 (m, 1H), 3.29 - 3.59 (m, 3H), 2.37 - 2.43(m, 1H), 1.94 - 1.99 (m, 1H), 1.48 (s, 9H).

### Step C: (S)-5-fluoro-2-(pyrrolidin-3-ylthio)pyridine hydrochloride

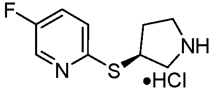

1.00 g (3.35 mmol, 1.0 eq) (*S*)-3-(5-fluoropyridin-2-yl)thio)pyrrolidine-1-tert-butyl carboxylate was dissolved in 20 mL dichloromethane, and then 8.4 mL (33.5 mmol, 10.0 eq) 4 M hydrochloric acid 1,4-dioxane solution was added to the above reaction system. The reaction system was stirred under nitrogen protection at room temperature for 1h. After confirming that the raw materials had reacted completely through TLC Plate (petroleum ether: ethyl acetate=10:1), the reaction solution was directly concentrated under reduced pressure to obtain a nearly white solid (0.66 g, yield=99%).

### Step D: (S)-5-bromo-2-(3-(5-fluoropyridin-2-yl)thio)pyrrolidin-1-yl)benzaldehyde

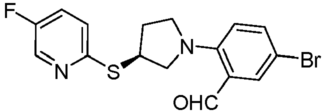

0.66 g (3.32 mmol, 1.0 eq) (*S*)-5-fluoro-2-(pyrrolidin-3-ylthio)pyridine hydrochloride was dissolved in 10 mL anhydrous *N*, *N*-dimethylformamide, and then 0.67 g (3.32 mmol, 1.0 eq) 5-bromo-2-fluorobenzaldehyde and 1.38 g (9.98 mmol, 3.0 eq) anhydrous potassium carbonate were added to the above reaction system. The reaction system reacted overnight under nitrogen protection at 90 °C. After confirming that the raw materials had been completely converted and the target product had been generated through LC-MS results, the reaction solution was cooled to room temperature and quenched by adding water. It was extracted three times with ethyl acetate. The organic phases were washed with salt water, dried over anhydrous sodium sulfate and concentrated to dryness, and then subjected to column chromatography (petroleum ether: ethyl acetate=10:1) to obtain a light yellow oily substance (0.99 g, yield=78%).
LC-MS: (M+H)⁺; m/z= 381.08;

### Step E: (S)-2'-cyclopropyl-4-(3-((5-fluoropyridin-2-yl)thio)pyrrolidin-1-yl)-[1,1'-biphenyl]-3-formaldehyde

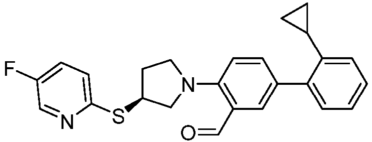

0.99 g (2.60 mmol, 1.0 eq) (*S*)-5-bromo-2-(3-(5-fluoropyridin-2-yl)thio)pyrrolidin-1-yl)benzaldehyde was dissolved in 20 mL dioxane and 5 mL water, and then 0.50 g (3.11 mmol, 1.2 eq) (2-cyclopropylphenyl)boric acid and 2.20 g (10.4 mmol, 4.0 eq) anhydrouspotassium phosphate were added to the above reaction system. 20 mg (0.026 mmol, 0.01 eq.) [1,1'-bis(diphenylphosphine)ferrocene] dichloride palladium was added under nitrogen protection and reacted at 100 °C for 4 hours. After confirming that the target product had been generated through LC-MS results, the reaction solution was cooled to room temperature and quenched by adding water. It was extracted twice with ethyl acetate. The organic phases were washed with salt water, dried over anhydrous sodium sulfate and concentrated to dryness, and then subjected to column chromatography (petroleum ether: ethyl acetate=10:1) to obtain a light yellow oily substance (900 mg, yield=82%).
LC-MS: (M+H)⁺; m/z= 419.10;
¹H NMR (400 MHz, CDCl₃) δ 10.13 (s, 1H), 8.34 (d, *J =* 4.0 Hz, 1H), 7.83 (d, *J =* 4.0 Hz, 1H), 7.59 (dd, *J =* 4.0 Hz, 4.0 Hz, 1H),7.32 - 7.18 (m, 5H), 6.98 - 6.93 (m, 2H), 4.42 - 4.50 (m, 1H), 4.02 (d, *J =* 8.0 Hz, 1H), 3.61 - 3.68 (m, 2H), 3.48 (d, *J =* 8.0 Hz, 1H), 2.55 - 2.62 (m, 1H), 2.16 - 2.22 (m, 1H), 1.90 - 1.94 (m, 1H), 0.86 - 0.92 (m, 2H), 0.77 - 0.75 (m, 2H).

### Step F: ((S)-(4-(3-(5-fluoropyridin-2-yl)thio)pyrrolidin-1-yl)-2'-cyclopropyl-[1,1'-biphenyl]-3-yl)methanol

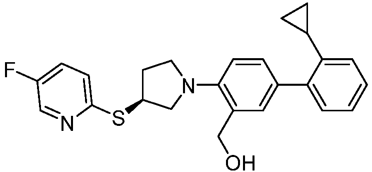

900 mg (2.15 mmol, 1.0 eq) ((*S*)-2'-cyclopropyl-4-(3-((5-fluoropyridin-2-yl)thio)pyrrolidin-1-yl)-[1,1'-biphenyl]-3-formaldehyde was dissolved in 10 mL methanol, and the system was cooled to 0 °C. 163 mg (4.30 mmol, 2.0 eq) sodium borohydride solid was added to the above reaction system under nitrogen protection. After the addition, the reaction system was naturally heated to room temperature and reacted for 1.5 h. After confirming that the target product had been generated through LC-MS results, the reaction solution was cooled to 0 °C and quenched by adding saturated ammonium chloride aqueous solution. It was extracted three times with ethyl acetate. The organic phases were washed with salt water, dried over anhydrous sodium sulfate and concentrated to dryness. The sample was sent for preparation and purification (mobile phase A: 0.1% formic acid; mobile phase B: acetonitrile; gradient: 30% -70% B, 55 min; flow rate: 70mL/min), and then freeze-dried to obtain a yellow oily product (667 mg, yield=73%).
LC-MS: (M+H)⁺; m/z= 421.16;
¹H NMR (400 MHz, CDCl₃) δ 8.34 (d, *J =* 4.0 Hz, 1H), 7.17 - 7.37 (m, 8H), 6.92 - 6.94 (m, 1H), 4.89 - 4.81 (m, 2H), 4.41 - 4.47 (m, 1H), 4.25 (s, 1H), 3.80 (d, *J =* 4.0 Hz, 1H), 3.46 - 3.50 (m, 1H), 3.29 - 3.35 (m, 2H), 2.56 - 2.64 (m, 1H), 2.05 - 2.13 (m, 1H), 1.88 - 1.95 (m, 1H), 0.84 - 0.89 (m, 2H), 0.70 - 0.74 (m, 2H).

Examples in Table 1 were prepared by using the method described in Example 1 above, but replacing the starting materials with (*S*)-1-BOC-3-hydroxypyrrolidine, 1-BOC-3-hydroxypyrrolidine, and other required materials, respectively.

**Table 1: Examples 11-14**

| Exam ple No. | Structure | Compound Name | Spectral Data |
|---|---|---|---|
| 11 | 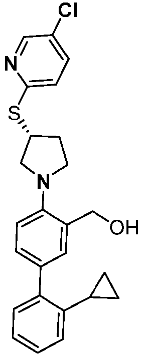 | ((*R*)-(4-(3-(5-chloropyridin-2-yl)thio)pyrrolidin-1-yl)-2'-cyclopropyl-[1,1'-biphenyl]-3-yl)methanol | LC-MS: (M+H)⁺; m/z= 437.31; |
| | | | ¹H NMR (400 MHz, CDCl3) δ 8.41 (d, *J* = 4.0 Hz, 1H), 7.469 (dd, *J* = 8.0 Hz, 4.0 Hz, 1H), 7.37 - 7.33 (m, 2H),7.28 - 7.25 (m, 1H), 7.23 - 7.20 (m, 2H), 7.18 - 7.15 (m, 2H), 6.93 (d, *J =* 8.0 Hz, 1H), 4.85 - 4.76 (m, 2H), 4.49 - 4.44 (m, 1H), 4.21 (s, 1H), 3.80 - 3.78 (m, 1H), 3.52 - 3.46 (m, 1H) , 3.35 - 3.27 (m, 2H), 2.65 - 2.57 (m, 1H), 2.13 - 2.05 (m, 1H), 1.95 - 1.88 (m, 1H), 0.89 - 0.84 (m, 2H), 0.75 - 0.71 (m, 2H). |
| 12 | 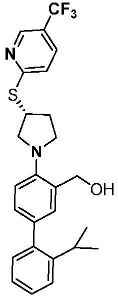 | ((*R*)-(2'-isopropyl-4-(3-(5-(trifluoromethyl)pyridin-2-yl)thio)pyrrolidin-1-yl)-[1,1'-biphenyl]-3-yl)methanol | LC-MS: (M+H)⁺; m/z= 473.21; |
| | | | ¹H NMR (400 MHz, CDCl3) δ 8.67 (s, 1H), 7.68 (d, *J =* 8.0 Hz, 1H), 7.38 - 7.27 (m, 3H), 7.21 - 7.13 (m, 5H), 4.85 - 4.77 (m, 2H), 4.56 - 4.50 (m, 1H), 4.14 (s, 1H), 3.82 - 3.78 (m, 1H), 3.51-3.45 (m, 1H), 3.35 - 3.26 (m, 2H), 3.10 - 3.03 (m, 1H), 2.66- 2.58 (m, 1H), 2.14- 2.05 (m, 1H), 1.15 (d, *J* = 4.0 Hz, 6H). |
| 13 | 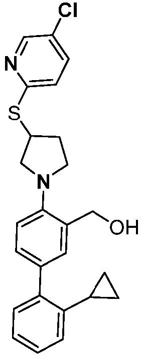 | (4-(3-(5-chloropyridin-2-yl)thio)pyrrolidin-1-yl)-2'-cyclopropyl-[1,1'-biphenyl]-3-yl)methanol | LC-MS: (M+H)⁺; m/z= 437.28 ; |
| | | | ¹H NMR (400 MHz, CDCl3) δ 8.41 (d, *J* = 4.0 Hz, 1H), 7.46 (dd, *J* = 8.0 Hz, 4.0 Hz, 1H), 7.37 - 7.33 (m, 2H),7.28 - 7.25 (m, 1H), 7.23 - 7.20 (m, 2H), 7.18 - 7.15 (m, 2H), 6.93 (d, *J =* 8.0 Hz, 1H), 4.85 -4.76 (m, 2H), 4.49 - 4.44 (m, 1H), 4.21 (s, 1H), 3.80 - 3.76 (m, 1H), 3.52 - 3.46 (m, 1H) , 3.35 - 3.27 (m, 2H), 2.65 - 2.57 (m, 1H), 2.13 - 2.05 (m, 1H), 1.95 - 1.88 (m, 1H), 0.89 - 0.84 (m, 2H), 0.75 - 0.71 (m, 2H). |
| 14 | 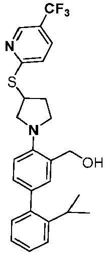 | (2'-isopropyl-4-(3-(5-(trifluoromethyl)pyridin-2-yl)thio)pyrrolidin-1-yl)-[1,1'-biphenyl]-3-yl)methanol | LC-MS: (M+H)⁺; m/z= 473.22 ; |
| | | | ¹H NMR (400 MHz, CDCl3) δ 8.67 (s, 1H), 7.68 (d, *J =* 8.0 Hz, 1H), 7.38 - 7.27 (m, 3H), 7.21 - 7.13 (m, 5H), 4.84 - 4.75 (m, 2H), 4.56 - 4.50 (m, 1H), 4.14 (s, 1H), 3.82 - 3.78 (m, 1H), 3.51-3.45 (m, 1H), 3.35 - 3.26 (m, 2H), 3.10 - 3.03 (m, 1H), 2.66- 2.58 (m, 1H), 2.14- 2.05 (m, 1H), 1.15 (d, *J* = 4.0 Hz, 6H). |

Examples in Table 2 were prepared by using the method described in Example 1 above, but replacing with desired different substituted sulfhydryl raw materials.

**Table 2: Examples 15-33**

| Examp le No. | Structure | Compound Name | Spectral Data |
|---|---|---|---|
| 15 | 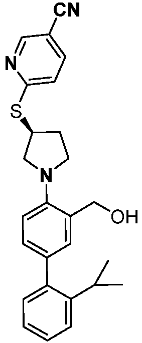 | (*S*)-6-((1-(3-(hydroxymethyl)-2'-isopropyl-[1,1'-biphenyl]-4-yl)pyrrolidin-3-yl)thio)nicotinonitrile | LC-MS: (M+H)⁺; m/z= 430.30 |
| 16 | 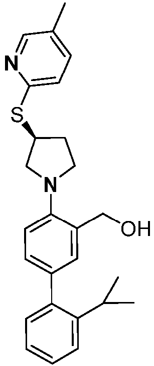 | (*S*)-(2'-isopropyl-4-(3-((5-methylpyridin-2-yl)thio)pyrrolidin-1-yl)-[1,1'-biphenyl]-3-yl)methanol | LC-MS: (M+H)⁺; m/z= 419.20 |
| 17 | 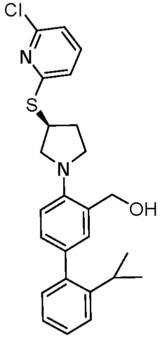 | (*S*)-(4-(3-((6-chloropyridin-2-yl)thio)pyrrolidin-1-yl)-2'-isopropyl-[1,1'-biphenyl]-3-yl)methanol | LC-MS: (M+H)⁺; m/z= 439.10 |
| 18 | 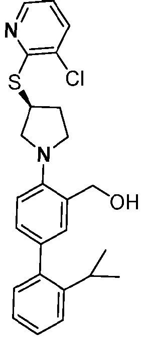 | (*S*)-(4-(3-((3-chloropyridin-2-yl)thio)pyrrolidin-1-yl)-2'-isopropyl-[1,1'-biphenyl]-3-yl)methanol | LC-MS: (M+H)⁺; m/z= 439.15 |
| 19 | 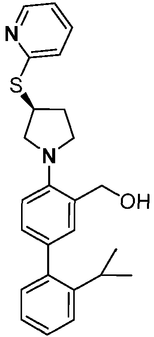 | (*S*)-(2'-isopropyl-4-(3-(pyridin-2-ylthio)pyrrolidin-1-yl)-[1,1'-biphenyl]-3-yl)methanol | LC-MS: (M+H)⁺; m/z= 405.18 |
| 20 | 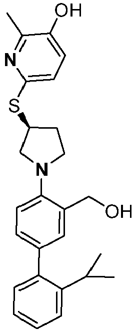 | (*S*)-6-((1-(3-(hydroxymethyl)-2'-isopropyl-[1,1'-biphenyl]-4-yl)pyrrolidin-3-yl)thio)- 2-methylpyridin-3-ol | LC-MS: (M+H)⁺; m/z= 435.19 |
| 21 | 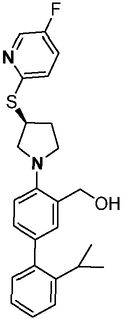 | (*S*)-(4-(3-((5-fluoropyridin-2-yl)thio)pyrrolidin-1-yl)-2'-isopropyl-[1,1'-biphenyl]-3-yl)methanol | LC-MS: (M+H)⁺; m/z= 422.17 |
| 22 | 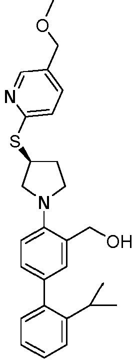 | (*S*)-(2'-isopropyl-4-(3-((5-(methoxymethyl)pyridin-2-yl)thio)pyrrolidin-1-yl)-[1,1'-biphenyl]-3-yl)methanol | LC-MS: (M+H)⁺; m/z= 449.23 |
| 23 | 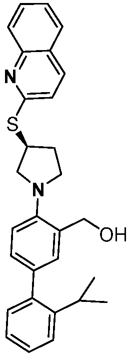 | (*S*)-(2'-isopropyl-4-(3-(quinolin-2-ylthio)pyrrolidin-1-yl)-[1,1'-biphenyl]-3-yl)methanol | LC-MS: (M+H)⁺; m/z=455.41; |
| | | | ¹H NMR (400 MHz, CDCl₃) δ 7.94 - 7.90 (m, 2H), 7.73 (d, *J* = 7.9 Hz, 1H), 7.67 - 7.63 (m, 1H), 7.46 - 7.42 (m, 1H), 7.40 - 7.28 (m, 3H), 7.21 - 7.12 (m, 5H), 4.87 - 4.79 (m, 2H), 4.73 - 4.68 (m, 1H), 3.94 - 3.90 (m, 1H), 3.53 - 3.45 (m, 1H), 3.41 - 3.29 (m, 2H), 3.08 - 3.05 (m, 1H), 2.72 - 2.65 (m, 1H), 2.24 - 2.12 (m, 2H), 1.15 (d, *J* = 6.9 Hz, 6H). |
| 24 | 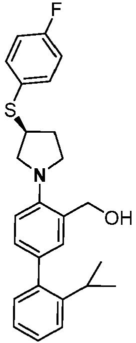 | (*S*)-(4-(3-((4-fluorophenyl)thio)pyrrolidin-1-yl)-2'-isopropyl-[1,1'-biphenyl]-3-yl)methanol | LC-MS:(M+H)⁺; m/z=422.21; |
| | | | ¹H NMR (400 MHz, CDCl₃) δ 7.47 - 7.44 (m, 2H), 7.39 - 7.31 (m, 2H), 7.21 - 7.10 (m, 5H), 7.05 - 7.01 (m, 2H), 4.85 - 4.75 (m, 2H), 4.29 (brs, 1H), 3.87 - 3.83 (m, 1H), 3.55 - 3.51 (m, 1H), 3.47 - 3.43 (m, 1H) , 3.26 - 3.21 (m, 2H), 3.08 - 3.05 (m, 1H), 2.46 - 2.42 (m, 1H), 2.02- 2.01 (m, 1H), 1.16 (d, *J* = 8.0 Hz, 6H). |
| 25 | 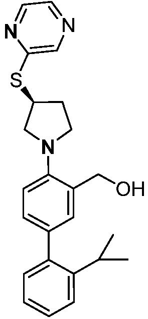 | (*S*)-(2'-isopropyl-4-(3-(pyrazin-2-ylthio)pyrrolidin-1-yl)-[1,1'-biphenyl]-3-yl)methanol | LC-MS: (M+H)⁺; m/z= 406.20 |
| 26 | 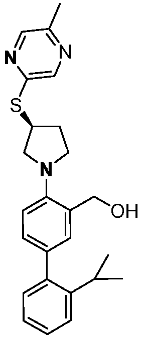 | (*S*)-(2'-isopropyl-4-(3-((5-methylpyrazin-2-yl)thio)pyrrolidin-1-yl)-[1,1'-biphenyl]-3-yl)methanol | LC-MS: (M+H)⁺; m/z= 420.22 |
| 27 | 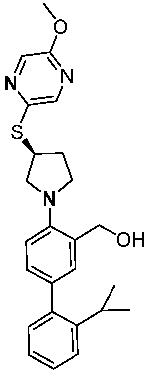 | (*S*)-(2'-isopropyl-4-(3-((5-methoxypyrazin-2-yl)thio)pyrrolidin-1-yl)-[1,1'-biphenyl]-3-yl)methanol | LC-MS: (M+H)⁺; m/z= 436.22 |
| 28 | 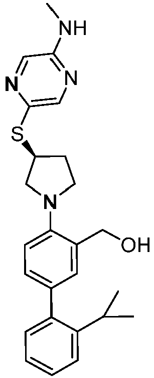 | (*S*)-(2'-isopropyl-4-(3-((5-(methylamino)pyrazin-2-yl)thio)pyrrolidin-1-yl)-[1,1'-biphenyl]-3-yl)methanol | LC-MS: (M+H)⁺; m/z= 435.19 |
| 29 | 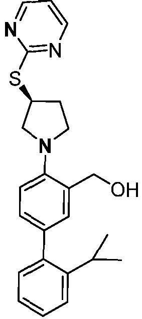 | (*S*)-(2'-isopropyl-4-(3-(pyrimidin-2-ylthio)pyrrolidin-1-yl)-[1,1'-biphenyl]-3-yl)methanol | LC-MS: (M+H)⁺; m/z= 406.17 |
| 30 | 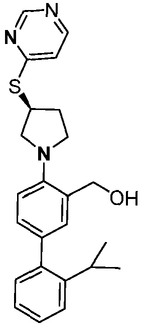 | (*S*)-(2'-isopropyl-4-(3-((5-methylpyrazin-2-yl)thio)pyrrolidin-1-yl)-[1,1'-biphenyl]-3-yl)methanol | LC-MS: (M+H)⁺; m/z= 406.19 |
| 31 | 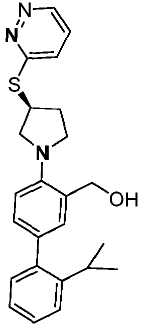 | (S)-(2'-isopropyl-4-(3-(pyridazin-3-ylthio)pyrrolidin-1-yl)-[1,1'-biphenyl]-3-yl)methanol | LC-MS: (M+H)⁺; m/z= 406.17 |
| 32 | 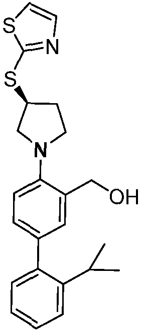 | (*S*)-(2'-isopropyl-4-(3-(thiazol-2-ylthio)pyrrolidin-1-yl)-[1,1'-biphenyl]-3-yl)methanol | LC-MS: (M+H)⁺; m/z= 411.17 |
| 33 | 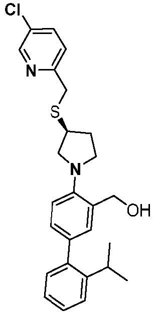 | (*S*)-(4-(3-(((5-chloropyridin-2-yl) methyl)thio)pyrrolidin-1-yl)-2'-isopropyl-[1,1'-biphenyl]-3-yl)methanol | LC-MS: (M+H)⁺; m/z= 453.16 |

Examples in Table 3 were prepared by using the method described in Example 1 above, except that Intermediate I3 and the desired different boronic acids or borates were prepared by Suzuki reaction.

**Table 3: Examples 34-40**

| Exam ple No. | Structure | Compound Name | Spectral Data |
|---|---|---|---|
| 34 | 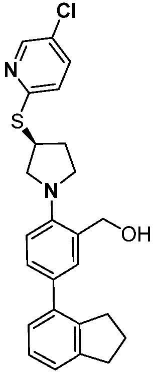 | (*S*)-(2-(3-((5-chloropyridin-2-yl)thio)pyrrolidin-1-yl)-5-(2,3-dihydro-1H-inden-4-yl) phenyl)methanol | LC-MS: (M+H)⁺; m/z=437.13; |
| | | | ¹H NMR (400 MHz, CDCl₃) δ 8.38 (d, *J* = 2.5 Hz, 1H), 7.46 (dd, *J* = 8.6, 2.6 Hz, 1H), 7.32 (d, *J* = 7.6 Hz, 2H), 7.21 - 7.20 (m, 2H), 7.18 - 7.13 (m, 3H), 4.88 - 4.75 (m, 2H), 4.44 - 4.41 (m, 1H), 3.78 - 3.74 (m, 1H), 3.49 - 3.43 (m, 1H), 3.32 - 3.25 (m, 2H), 2.99 - 2.94 (m, 4H), 2.64 - 2.52 (m, 1H), 2.09 - 2.00 (m, 4H). |
| 35 | 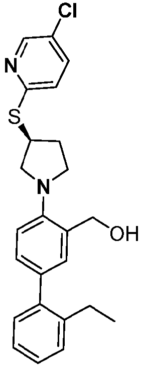 | (*S*)-(4-(3-((5-chloropyridin-2-yl)thio)pyrrolidin-1-yl)-2'-ethyl-[1,1'-biphenyl]-3-yl)methanol | LC-MS: (M+H)⁺; m/z=425.14; |
| | | | ¹H NMR (400 MHz, CDCl₃) δ 8.43 (s, 1H), 7.52 (d, *J* = 8.6 Hz, 1H), 7.34 - 7.31 (m, 3H), 7.24 - 7.18 (m, 5H), 4.90 - 4.81 (m, 2H), 4.48 (s, 1H), 3.83 - 3.79 (m, 1H), 3.54 - 3.48 (m, 1H), 3.37 - 3.29 (m, 2H), 2.67 - 2.59 (m, 3H), 2.03 - 2.01 (m, 2H), 1.15 (t,*J* = 7.5 Hz, 3H). |
| 36 | 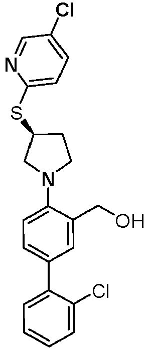 | (*S*)-(2'-cloro-4-(3-((5-chloropyridin-2-yl)thio)pyrrolidin-1-yl)-[1,1'-biphenyl]-3-yl)methanol | LC-MS: (M+H)⁺; m/z=431.03; |
| | | | ¹H NMR (400 MHz, CDCl₃) δ 8.38 (d, *J* = 2.5 Hz, 1H), 7.54 - 7.39 (m, 2H), 7.35 - 7.27 (m, 5H), 7.15 - 7.11 (m, 2H), 4.96 - 4.70 (m, 2H), 4.46 - 4.40 (m, 1H), 3.92 - 3.71 (m, 2H), 3.52 - 3.46 (m, 1H), 3.35 - 3.28 (m, 2H), 2.67 - 2.50 (m, 1H), 2.11 - 2.03 (m, 1H). |
| 37 | 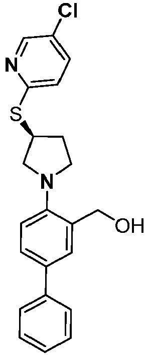 | (*S*)-(4-(3-((5-chloropyridin-2-yl)thio)pyrrolidin-1-yl)-[1,1'-biphenyl]-3-yl)methanol | LC-MS: (M+H)⁺; m/z= 397.12 |
| 38 | 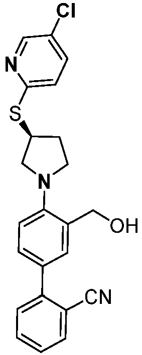 | (*S*)-4'-(3-((5-chloropyridin-2-yl)thio)pyrrolidin-1-yl)-3'-(hydroxymethyl)-[1,1'-biphenyl]-2- carbonitrile | LC-MS: (M+H)⁺; m/z=422.06; |
| | | | ¹H NMR (400 MHz, CDCl₃) δ 8.39 (d, *J* = 2.5 Hz, 1H), 7.74 (d, *J* = 7.7 Hz, 1H), 7.67 - 7.58 (m, 1H), 7.53 - 7.44 (m, 4H), 7.41 - 7.38 (m, 1H), 7.15 - 7.10 (m, 2H), 4.93 - 4.73 (m, 2H), 4.46 - 4.40 (m, 1H), 3.85 - 3.81 (m, 1H), 3.55 - 3.49 (m, 1H), 3.38 - 3.33 (m, 3H), 2.66 - 2.49 (m, 1H), 2.12 - 2.04 (m, 1H). |
| 39 | 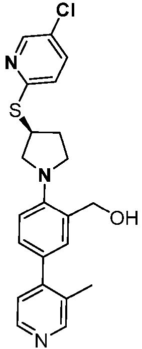 | (*S*)-(2-(3-((5-chloropyridin-2-yl)thio)pyrrolidin-1-yl)-5-(3-methylpyridin-4-yl)phenyl)methanol | LC-MS: (M+H)⁺; m/z=412.09; |
| | | | ¹H NMR (400 MHz, CDCl₃) δ 8.48 (s, 1H), 8.44 (d, *J* = 5.1 Hz, 1H), 8.39 (d, *J* = 2.5 Hz, 1H), 7.47 (dd, *J* = 8.6, 2.6 Hz, 1H), 7.24 - 7.19 (m, 2H), 7.16 - 7.09 (m, 3H), 4.86 - 4.76 (m, 2H), 4.35 (brs, 1H), 3.82 - 3.78 (m, 1H), 3.53 - 3.46 (m, 1H), 3.35 - 3.29 (m, 2H), 2.60 - 2.54 (m, 1H), 2.30 (s, 3H), 2.12 - 2.04 (m, 2H). |
| 40 | 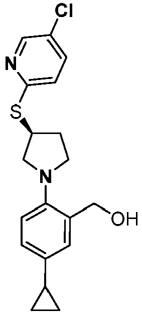 | (*S*)-(2-(3-((5-chloropyridin-2-yl)thio)pyrrolidin-1-yl)-5-cyclopropyl phenyl)methanol | LC-MS: (M+H)⁺; m/z=361.12; |
| | | | ¹H NMR (400 MHz, CDCl₃) δ 8.36 (d, *J* = 2.5 Hz, 1H), 7.45 (dd, *J* = 8.6, 2.5 Hz, 1H), 7.12 (d, *J* = 8.6 Hz, 1H), 7.05 (d, *J* = 8.2 Hz, 1H), 6.95 (dd, *J =* 8.2, 2.2 Hz, 1H), 6.90 (d, *J =* 2.2 Hz, 1H), 4.79 - 4.69 (m, 2H), 4.40 - 4.36 (m, 1H), 3.66 - 3.61 (m, 1H), 3.40 - 3.26 (m, 1H), 3.19 - 3.14 (m, 2H), 2.64 - 2.48 (m, 1H), 2.03 - 2.00 (m, 1H), 1.86 - 1.82 (m, 1H), 0.94 - 0.86 (m, 3H), 0.71 - 0.58 (m, 2H). |

### Example 41: (S)-(3-(3-((5-chloropyridin-2-yl)thio)pyrrolidin-1-yl)-2'-isopropyl-[1,1'-biphenyl]-4-yl)methanol

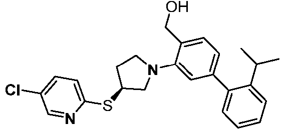

### Step A: (S)-4-bromo-2-(3-((5-chloropyridin-2-yl)thio)pyrrolidin-1-yl)benzaldehyde

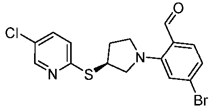

140 mg (652 umol, 1.0 eq) (S)-5-chloro-2-(pyrrolidin-3-ylthio)pyridine was dissolved in 5mL *N, N-*dimethylformamide, and 198 mg (978 umol, 1.5 eq) 4-bromo-2-fluorobenzaldehyde and 180 mg (1.3 mmol, 2.0 eq) potassium carbonate were added. Nitrogen replacement was carried out for three times, and stirring was carried out overnight at 100 °C. After confirming that the product had been generated through LC-MS, 5mL water was added thereto, and then extraction was carried out with ethyl acetate for three times. The organic layer was washed with 5 mL saturated saline solution, dried over anhydrous sodium sulfate and evaporated under reduced pressure. The residue was subjected to column chromatography (dichloromethane/methanol=0~15:1) to obtain the product (190 mg, yield=73%).
LC-MS: (M+H)⁺; m/z=398.84;

### Step B: (S)-3-(3-((5-chloropyridin-2-yl)thio)pyrrolidin-1-yl)-2'-isopropyl-[1,1'-biphenyl]-4-formaldehyde

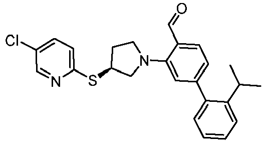

50.0 mg (126 umol, 1.0 eq) (*S*)-4-bromo-2-(3-((5-chloropyridin-2-yl)thio)pyrrolidin-1-yl)benzaldehyde was dissolved in 4 mL dioxane and 1 mL water, and then 24.7 mg (151 umol, 1.2 eq) 2-isopropylphenylboronic acid, 9.20 mg (13 umol, 0.1 eq) 1,1-bis(diphenylphosphine) ferrocene dichloride palladium and 52.1 mg (377 umol, 3.0 eq) potassium carbonate were added. Nitrogen replacement was carried out for three times, and stirring was carried out at 100 °C for 2 h. After confirming that the product had been generated through LC-MS, 5mL water was added thereto, and then extraction was carried out with ethyl acetate for three times. The organic layer was washed with 5 mL saturated saline solution, dried over anhydrous sodium sulfate and evaporated under reduced pressure to obtain 48.0 mg of a light yellow oily liquid which was used directly in the subsequent reaction without purification.
LC-MS: (M+H)⁺; m/z=437.05;

### Step C: (S)-3-(3-((5-chloropyridin-2-yl)thio)pyrrolidin-1-yl)-2'-isopropyl-[1,1'-biphenyl]-4-methanol

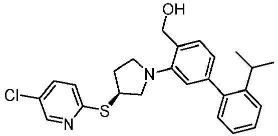

48.0 mg (110 umol, 1.0 eq) (*S*)-3-(3-((5-chloropyridin-2-yl)thio)pyrrolidin-1-yl)-2'-isopropyl-[1,1'-biphenyl]-4-formaldehyde was dissolved in 5mL methanol. In an ice bath, 8.32 mg (220 umol, 2.0 eq) sodium borohydride was slowly added. Stirring was carried out at room temperature for 10 min. After confirming that the product had been generated through LC-MS, evaporation was carried out under reduced pressure. Preparation and Purification were carried out (mobile phase A: 0.1% formic acid; mobile phase B: acetonitrile; gradient: 30% -70% B, 55 min; flow rate: 70mL/min) to obtain a white solid (20.9 mg, two-step yield=38%).
LC-MS: (M+H)⁺; m/z=439.36;
¹H NMR (400 MHz, CDCl₃) δ 8.36 (d, *J* = 2.3 Hz, 1H), 7.45 (dd, *J =* 8.5, 2.6 Hz, 1H), 7.42 - 7.31 (m, 2H), 7.25 (d, *J =* 7.8 Hz, 1H), 7.23 - 7.09 (m, 3H), 7.02 - 6.91 (m, 2H), 4.90 - 4.76 (m, 2H), 4.41 - 4.40 (m, 1H), 3.99 (s, 1H), 3.75 - 3.71 (m, 1H), 3.45 - 3.43 (m, 1H), 3.30 - 3.24 (m, 2H), 3.05 - 3.02 (m, 1H), 2.56 - 2.54 (m, 1H), 2.05 - 2.03 (m, 1H), 1.16 (d, *J =* 6.7 Hz, 6H).

Examples in Table 4 were prepared by using the general scheme 2 or scheme 3 described above.

**Table 4: Examples 42-76**

| Exa mple No. | Structure | Compound Name | Spectral Data |
|---|---|---|---|
| 42 | 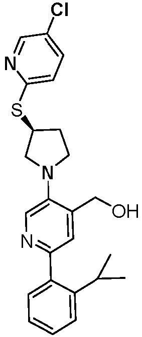 | (*S*)-(5-(3-((5-chloropyridin-2-yl)thio)pyrrolidin-1-yl)-2-(2-isopropylphenyl)pyridin4-yl)methanol | LC-MS: (M+H)⁺; m/z= 440.10 |
| 43 | 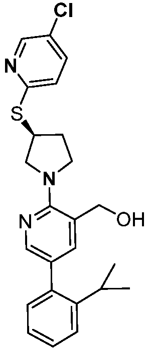 | (*S*)-(2-(3-((5-chloropyridin-2-yl)thio)pyrrolidin-1-yl)-5-(2-isopropylphenyl)pyridin3-yl)methanol | LC-MS: (M+H)⁺; m/z= 440.16 |
| 44 | 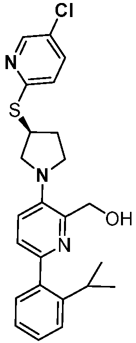 | (*S*)-(3-(3-((5-chloropyridin-2-yl)thio)pyrrolidin-1-yl)-6-(2-isopropylphenyl)pyridin-2-methyl)methanol | LC-MS: (M+H)⁺; m/z= 440.16 |
| 45 | 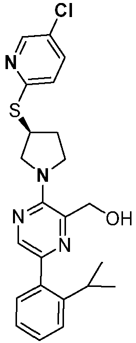 | (*S*)-(3-(3-((5-chloropyridin-2-yl)thio)pyrrolidin-1-yl)-6-(2-isopropyl phenyl)pyrazin-2-yl | LC-MS: (M+H)⁺; m/z= 441.13 |
| 46 | 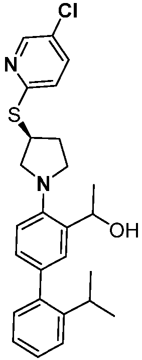 | 1-(4-((*S*)-3-((5-chloropyridin-2-yl)thio)pyrrolidin-1-yl)-2'-isopropyl-[1,1'-biphenyl]-3-yl)ethan-1-ol | LC-MS: (M+H)⁺; m/z= 453.16 |
| | | | ¹H NMR (400 MHz, CDCl₃) δ 8.37 (dd, *J* = 4.8, 2.5 Hz, 1H), 7.46 (ddd, *J* = 8.6, 4.9, 2.5 Hz, 1H), 7.39 - 7.32 (m, 2H), 7.30 - 7.27 (m, 1H), 7.22 - 7.12 (m, 5H), 5.93 (s, 1H), 5.20 - 5.10 (m, 1H), 4.49 - 4.39 (m, 1H), 3.84 - 3.62 (m, 1H), 3.58 - 3.30 (m, 2H), 3.20 - 3.08 (m, 1H), 3.06 - 2.98 (m, 1H), 2.65 - 2.56 (m, 1H), 2.07 - 2.05 (m, 1H), 1.55 (d, 3H), 1.16 (d, *J =* 6.9 Hz, 6H). |
| 47 | 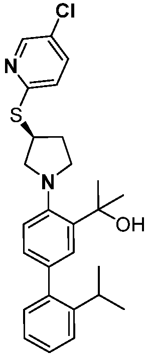 | (*S*)-2-(4-(3-((5-chloropyridin-2-yl)thio)pyrrolidin-1-yl)-2'-isopropyl-[1,1'-biphenyl]-3-yl)propanol | LC-MS: (M+H)⁺; m/z= 467.17 |
| 48 | 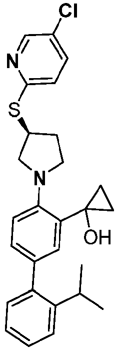 | (*S*)-1-(4-(3-((5-chloropyridin-2-yl)thio)pyrrolidin-1-yl)-2'-isopropyl-[1,1'-biphenyl]-3-yl)cyclopropane-1-ol | LC-MS: (M+H)⁺; m/z= 465.16 |
| 49 | 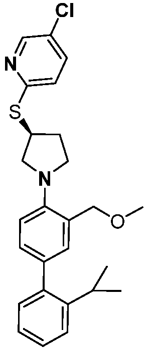 | (*S*)-5-chloro-2-((1-(2'-isopropyl-3-(methoxymethyl)-[1,1'-biphenyl]-4-yl)pyrrolidin-3-yl)thio)pyridine | LC-MS: (M+H)⁺; m/z= 453.18 |
| 50 | 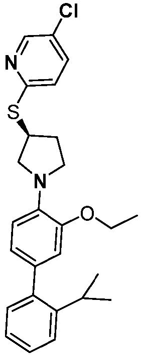 | (*S*)-5-chloro-2-((1-(3-ethoxy-2'-isopropyl-[1,1'-biphenyl]-4-yl)pyrrolidin-3-yl)thio)pyridine | LC-MS: (M+H)⁺; m/z= 453.08 |
| 51 | 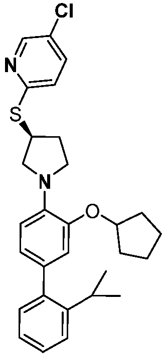 | (*S*)-5-chloro-2-((1-(3-(cyclopentyloxy)-2'-isopropyl-[1,1'-biphenyl]-4-yl)pyrrolidin-3-yl)thio) pyridine | LC-MS: (M+H)⁺; m/z= 493.17 |
| 52 | 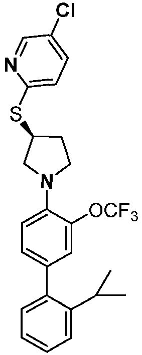 | (*S*)-5-chloro-2-((1-(2'-isopropyl-3-trifluoromethoxy)-[1,1'-biphenyl]-4-yl)pyrrolidin-3-yl)thio)pyridine | LC-MS: (M+H)⁺; m/z= 493.10 |
| 53 | 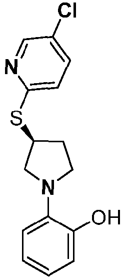 | (*S*)-2-(3-((5-chloropyridin-2-yl)thio)pyrrolidin-1-yl)phenol | LC-MS: (M+H)⁺; m/z= 307.04 |
| 54 | 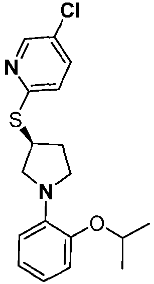 | (*S*)-5-chloro-2-((1-(2-isopropoxyphenyl)pyrrolidin-3-yl)thio)pyridine | LC-MS: (M+H)⁺; m/z= 349.09 |
| 55 | 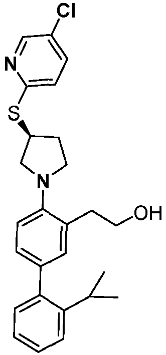 | (*S*)-2-(4-(3-((5-chloropyridin-2-yl)thio)pyrrolidin-1-yl)-2'-isopropyl-[1,1'-biphenyl]-3-yl)ethan-1-ol | LC-MS: (M+H)⁺; m/z= 453.15 |
| 56 | 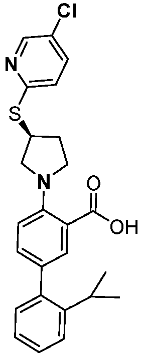 | (*S*)-4-(3-((5-chloropyridin-2-yl)thio)pyrrolidin-1-yl)-2'-isopropyl-[1,1'-biphenyl]-3-carboxylic acid | LC-MS: (M+H)⁺; m/z= 453.11 |
| 57 | 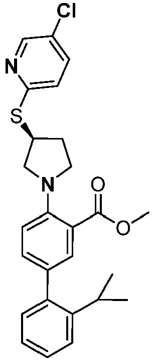 | methyl(*S*)-4-(3-((5-chloropyridin-2-yl)thio)pyrrolidin-1-yl)-2'-isopropyl-[1,1'-biphenyl]-3- carboxylate | LC-MS: (M+H)⁺; m/z= 467.13 |
| 58 | 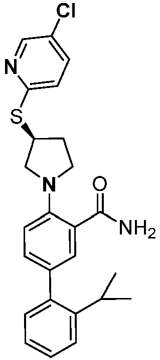 | (*S*)-4-(3-((5-chloropyridin-2-yl)thio)pyrrolidin-1-yl)-2'-isopropyl-[1,1'-biphenyl]-3-carboxamide | LC-MS: (M+H)⁺; m/z= 452.13 |
| 59 | 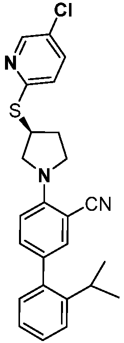 | (*S*)-4-(3-((5-chloropyridin-2-yl)thio)pyrrolidin-1-yl)-2'-isopropyl-[1,1'-biphenyl]-3- carbonitrile | LC-MS: (M+H)⁺; m/z= 434.11 |
| 60 | 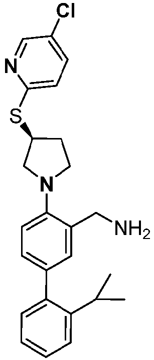 | (*S*)-(4-(3-((5-chloropyridin-2-yl)thio)pyrrolidin-1-yl)-2'-isopropyl-[1,1'-biphenyl]-3-yl)methylamine | LC-MS: (M+H)⁺; m/z= 438.16 |
| 61 | 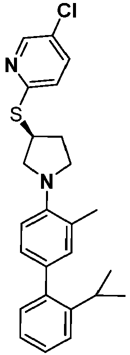 | (*S*)-5-chloro-2-((1-(2'-isopropyl-3-methyl-[1,1'-biphenyl]-4-yl)pyrrolidin-3-yl)thio)pyridine | LC-MS: (M+H)⁺; m/z= 423.14 |
| 62 | 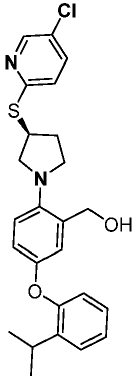 | (*S*)-(2-(3-((5-chloropyridin-2-yl)thio)pyrrolidin-1-yl)-5-(2-isopropylphenoxy) phenyl)methanol | LC-MS: (M+H)⁺; m/z= 455.13 |
| 63 | 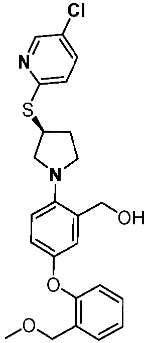 | (*S*)-(2-(3-((5-chloropyridin-2-yl)thio)pyrrolidin-1-yl)-5-(2-methoxymethyl)phenoxy) phenyl)methanol | LC-MS: (M+H)⁺; m/z= 457.11 |
| 64 | 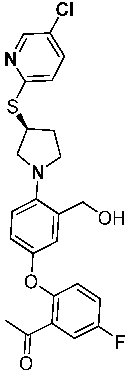 | (*S*)-1-(2-(4-(3-((5-chloropyridin-2-yl)thio)pyrrolidin-1-yl)-3-(hydroxymethyl)phenoxy)-5-fluorophenyl)ethan-1-one | LC-MS: (M+H)⁺; m/z= 473.08 |
| 65 | 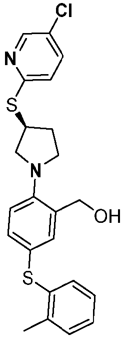 | (*S*)-(2-(3-((5-chloropyridin-2-yl)thio)pyrrolidin-1-yl)-5-(o-methylphenylthio)phenyl) methanol | LC-MS: (M+H)⁺; m/z= 443.07 |
| 66 | 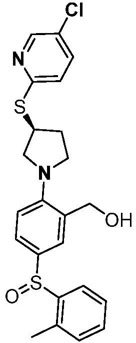 | (2-((*S*)-3-((5-chloropyridin-2-yl)thio)pyrrolidin-1-yl)-5-(o-toluenesulfonyl)phenyl)methanol | LC-MS: (M+H)⁺; m/z= 459.06 |
| 67 | 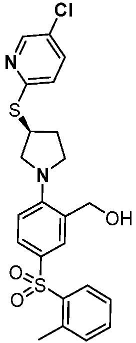 | (*S*)-(2-(3-((5-chloropyridin-2-yl)thio)pyrrolidin-1-yl)-5-(o-toluenesulfonyl)phenyl)methanol | LC-MS: (M+H)⁺; m/z= 475.07 |
| 68 | 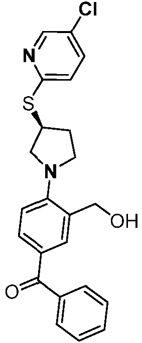 | (*S*)-(4-(3-((5-chloropyridin-2-yl)thio)pyrrolidin-1-yl)-3-(hydroxymethyl)phenyl)(phenyl)ketone | LC-MS: (M+H)⁺; m/z= 425.08 |
| 69 | 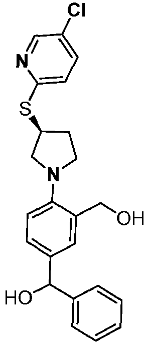 | (4-((*S*)-3-((5-chloropyridin-2-yl)thio)pyrrolidin-1-yl)-3-hydroxymethylphenyl)(phenyl)methanol | LC-MS: (M+H)⁺; m/z= 427.10 |
| 70 | 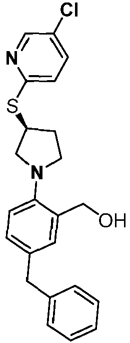 | (*S*)-(5-benzyl-2-(3-((5-chloropyridin-2-yl)thio)pyrrolidin-1-yl)phenyl)methanol | LC-MS: (M+H)⁺; m/z= 411.10 |
| 71 | 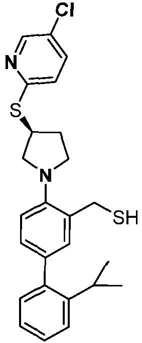 | (*S*)-(4-(3-((5-chloropyridin-2-yl)thio)pyrrolidin-1-yl)-2'-isopropyl-[1,1'-biphenyl]-3-yl)methyl mercaptan | LC-MS: (M+H)⁺; m/z= 455.11 |
| 72 | 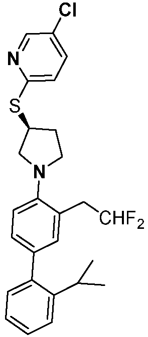 | (*S*)-5-chloro-2-((1-(3-(2,2-difluoroethyl)-2'-isopropyl-[1,1'-biphenyl]-4-yl)pyrrolidin-3-yl)thio)pyridine | LC-MS: (M+H)⁺; m/z= 473.14 |
| 73 | 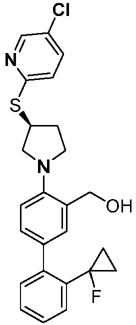 | (*S*)-(4-(3-((5-chloropyridin-2-yl)thio)pyrrolidin-1-yl)-2'-(1-fluorocyclopropyl)-[1,1'-biphenyl]-3-yl)methanol | LC-MS: (M+H)⁺; m/z =455.11 |
| 74 | 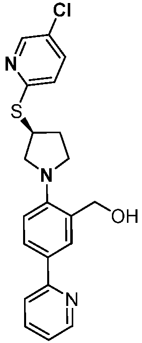 | (*S*)-(2-(3-((5-chloropyridin-2-yl)thio)pyrrolidin-1-yl)-5-(pyridin-2-)phenyl)methanol | LC-MS: (M+H)⁺; m/z= 398.08 |
| 75 | 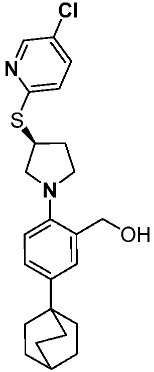 | (*S*)-(5-(bicyclo[2.2.2]octan-1-yl)-2-(3-((5-chloropyridin-2-yl)thio)pyrrolidin-1-yl)phenyl)methanol | LC-MS: (M+H)⁺; m/z= 429.16 |
| 76 | 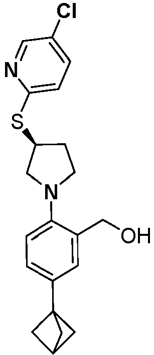 | (*S*)-(5-(bicyclo[1.1.1]pentan-1-yl)-2-(3-((5-chloropyridin-2-yl)thio)pyrrolidin-1-yl)phenyl)methanol | LC-MS: (M+H)⁺; m/z= 387.13 |

### Example 77 : (S)-5-chloro-2-((1-(3-(difluoromethyl)-2'-isopropyl-[1,1'-biphenyl]-4-yl)pyrrolidin-3-yl)thiopyridine

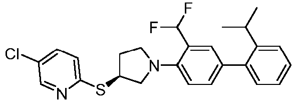

30.0 mg (68.7 µmol, 1.0 eq) (*S*)-4-(3-((5-chloropyridin-2-yl)thio)pyrrolidin-1-yl)-2'-isopropyl-[1,1'-biphenyl]-3-formaldehyde was dissolved in 0.5 mL anhydrous dichloromethane. After replacing with nitrogen, the system was cooled to 0 °C, and 16.6 mg (103 µ mol, 1.5 eq) diethylamino sulfur trifluoride was slowly added dropwise to the former. After adding dropwise, it was returned to room temperature naturally and continuously stirred for 18 h. After confirming complete reaction through LC-MS results, it was quenched by using 2mL cold saturated sodium bicarbonate solution, and then extracted three times with 5 mL dichloromethane. The organic phases were washed with saturated sodium chloride solution, dried over anhydrous sodium sulfate and evaporated under reduced pressure. The residue was subjected to preparative chromatography (mobile phase A: 0.1% trifluoroacetic acid; mobile phase B: acetonitrile; gradient: 30% -70% B, 55 min; flow rate: 70mL/min), and purified to obtain (*S*)-5-chloro-2-((1-(3-(difluoromethyl)-2'-isopropyl-[1,1'-biphenyl]-4-yl)pyrrolidin-3-yl)thio)pyridine as grey solid (4.3 mg, yield=14%).
LC-MS: (M+H)⁺; m/z=459;
¹H NMR (400 MHz, CDCl₃) *δ* 8.39 (d, *J =* 2.5 Hz, 1H), 7.53 (d, *J =* 2.1 Hz, 1H), 7.47 (dd, *J* = 8.5, 2.5 Hz, 1H), 7.39 - 7.29 (m, 3H), 7.22 - 7.16 (m, 2H), 7.13 - 7.05 (m, 2H), 6.98 - 6.91 (m, 1H), 4.44 - 4.41 (m, 1H), 3.85 - 3.85 (m, 1H), 3.48 - 3.47 (m, 1H), 3.39 (t, *J* = 7.4 Hz, 1H), 3.39 - 3.31 (m, 1H), 3.05 - 3.02 (m, 1H), 2.57 - 2.55 (m, 1H), 2.11 - 2.03 (m, 1H), 1.17 (d, *J*= 6.9 Hz, 6H).

### Example 78 : (S) -(2-(3-((5-chloropyridin-2-yl)thio)pyrrolidin-1-yl)-5-((2-isopropylphenyl) amino)phenyl)methanol

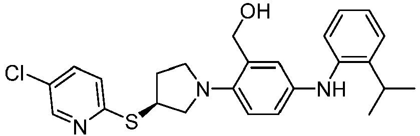

### Step A: (S) -2-(3-((5-chloropyridin-2-yl)thio)pyrrolidin-1-yl)-5-((2-isopropylphenyl) amino)benzaldehyde

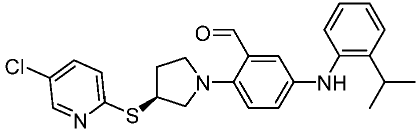

30.0 mg (75.4 µmol, 1.0e q) (*S*)-5-bromo-2-(3-((5-chloropyridin-2-yl)thio)pyrrolidin-1-yl)benzaldehyde was dissolved in 2 mL 1,4-dioxane, and then 11.2 mg (83.0 µmol, 1.1 eq) 2-isopropylaniline, 3.50 mg (3.77 µmol, 0.05 eq) tri(dibenzylideneacetone)dipalladium, 4.4 mg (7.54 µmol, 0.1 eq) 4,5-diphenylphosphine-9,9-dimethyloxaanthracene and 24.6 mg (75.4 µmol, 1.0 eq) cesium carbonate were added to the reaction system. After replacing with nitrogen, it was heated up to 100 °C and reacted for 5 h. After confirming complete reaction through LC-MS results, the sysem was returned to room temperature, and then diluted using 5mL saturated sodium chloride solution, and extracted three times with 10 mL ethyl acetate. The organic phases were taken for drying over anhydrous sodium sulfate and evaporated under reduced pressure. The residue was subjected to column chromatography (ethyl acetate: petroleum ether=1:10) to obtain (*S*)-2-(3-((5-chloropyridin-2-yl)thio)pyrrolidin-1-yl)-5-((2-isopropylphenyl) amino)benzaldehyde as a yellow oily liquid (8.30 mg, yield=24%).
LC-MS: (M+H)⁺; m/z=452;

### Step B: (S) -(2-(3-((5-chloropyridin-2-yl)thio)pyrrolidin-1-yl)-5-((2-isopropylphenyl) amino)phenyl)methanol

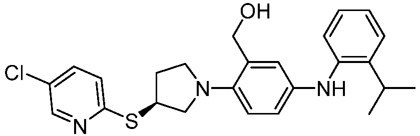

8.30 mg (18.4 µmol, 1.0 eq) (*S*)-2-(3-((5-chloropyridin-2-yl)thio)pyrrolidin-1-yl)-5-((2-isopropylphenyl)amino)benzaldehyde was dissolved in 0.5mL methanol. After replacing with nitrogen, the system was cooled to 0 °C, and then 0.80 mg (22.0 µmol, 1.2 eq) sodium borohydride was slowly added to the former. After the addition, it naturally returned to room temperature and continued to react for 30 minutes. After confirming complete reaction through LC-MS results, it was quenched by using 1mL saturated ammonium chloride solution, and then extracted three times with 5 mL dichloromethane. The organic phases were washed with saturated sodium chloride solution, dried over anhydrous sodium sulfate and evaporated under reduced pressure. The residue was subjected to preparative chromatography (mobile phase A: 0.1% trifluoroacetic acid; mobile phase B: acetonitrile; gradient: 30% -70% B, 55 min; flow rate: 70mL/min), and purified to obtain (*S*)-(2-(3-((5-chloropyridin-2-yl)thio)pyrrolidin-1-yl)-5-((2-isopropylphenyl)amino)phenyl)methanol as a yellow solid (1.0 mg, yield=12%).
LC-MS: (M+H)⁺; m/z=454.30;
¹H NMR (400 MHz, CDCl₃) *δ* 8.37 (d, *J* = 2.5 Hz, 1H), 7.45 (dd, *J* = 8.4, 2.4 Hz, 1H), 7.29 (d, *J* = 8.5 Hz, 1H), 7.23 - 6.98 (m, 5H), 6.82 - 6.69 (m, 2H), 5.34 (brs, 1H), 4.78 - 4.65 (m, 2H), 4.41 - 4.34 (m, 1H), 3.64 - 3.58 (m, 1H), 3.34 - 3.27 (m, 1H), 3.21 - 3.02 (m, 3H), 2.61 - 2.53 (m, 1H), 2.10 - 1.87 (m, 2H), 1.24 (d, *J =* 6.9 Hz, 6H).

### Biological activity testing of the compounds of the present invention

In this study, HEK-293 cells instantaneously expressing TRPV3 were used for experimental detection.

The operation steps are as follows:
The cells were cultured in DMEM medium containing 10% fetal bovine serum at a temperature of 37 °C and a carbon dioxide concentration of 5%.

Day 1: Cells were inoculated into a 6-well plate with 5 × 10⁵ cells per well.

Day 2: Transfection was performed using Lipofectamine 3000 transfection reagent, with a plasmid to transfection reagent ratio of 1 µg: 2 µL. The total amount of plasmid used per well was 3 µg. Specifically, two sterile centrifuge tubes were taken and 100 µL Opti-MEM was added to each tube. 6 µL Lipofectamine 3000 was added to one tube and mixed well; and 3 µg plasmid was added to another tube and mixed well, and then 6 µL P3000 was added and mixed well; after that, the diluted plasmid DNA was addeds to diluted Lipofectamine 3000 and incubated at room temperature for 10-15 min. DNA liposome complex was droped into the cells, shaked gently and mixed well, and then cultured in an incubator. The liquid was changeed after 4 to 6 h.

Day 3: The cells were digested and seeded into 24 well plates with pre-placed cover slides, with 8 × 10³ cells per well.

Day 4: Patch clamp testing was performed.

The voltage stimulation protocol for recording TRPV3 current using whole cell patch clamp is as follows: after forming a whole cell seal, a cell membrane voltage clamp was set at -80mV. Firstly, a membrane potential was recorded at 0mV, and then a voltage was command to start from -100mV, to depolarize in the form of Ramp to 100mV within 100ms, and finally restore to 0mV. Data collection was repeated every 5 seconds to observe the inhibitory effect of pharmaceutical on peak current. Experimental data was collected by EPC 10 amplifier (HEKA) and stored in PatchMaster (HEKA) software.

A microelectrode puller was used to pull the capillary glass tube into a recording electrode. The electrode filled with intracellular fluid was inserted into a microelectrode holder, and a microelectrode manipulator was operated under an inverted microscope to immerse the electrode in extracellular fluid and an electrode resistance (Rpip) was recorded. The electrode was brought into contact with the cell surface and a negative pressure suction was applied to form a high resistance seal (GΩ). At this point, a fast capacitance compensation was performed, and negative pressure was continued to apply in order to break the cell membrane, so that a whole cell recording mode was formed. Then, a slow capacitance compensation was performed and experimental parameters such as film capacitance (Cm) and series resistance (Rs) were recorded. No leakage compensation was provided.

When the TRPV3 current recorded by the whole cell had stabilized, administration was started. Each pharmaceutical concentration acted for 5 minutes (or until the current stabilized) before detecting the next concentration. A plurality of concentrations was tested for each test compound. A cover glass covered with cells was placed in a recording bath under an inverted microscope. A blank control extracellular solution and a working fluid for the compound to be tested sequentially flowed through the recording bath from low concentration to high concentration using gravity perfusion to act on the cells. Liquid exchange was performed using a peristaltic pump during recording. The current detected by each cell in the extracellular fluid without the compound serveed as its own control group. Each concentration was independently tested twice using at least two cells. All electrophysiological tests were conducted at room temperature.

The results obtained from testing the prepared compounds using the above analysis process are shown in Table 5. The details of the inhibition rate (%) at a concentration of 0.3 µM for the selected Examples are shown in the table. The thioether compounds of the present invention patent have soft pharmaceutical properties, while sulfoxides and sulfone metabolites do not have pharmacological activity (Table 5), so that it can be achieved that the toxicity of prototype drugs, active metabolites and reactive metabolites to a body is avoided while exerting therapeutic effects. Meanwhile, the compounds of the present invention patent also exhibit good selectivity (Table 6).

**Table 5: Inhibition rate (%) of the compounds of the present invention on hTRPV3 at a single concentration (0.3 µ M)**

| \| Example No. | Inhibition \| rate | Example No. | Inhibition rate | Example No. | Inhibition rate |
|---|---|---|---|---|---|
| 1 | 71.60 | 2 | 18.64 | 3 | 9.96 |
| 4 | 72.80 | 5 | 23.14 | 6 | 4.73 |
| 7 | 37.96 | 10 | 97.83 | KM-001^{a} | 67.15 |

| | | | | | |
|---|---|---|---|---|---|
| Note ^{a}: Example compound KM-001 in patent WO2021154966A1. | | | | | |

**Table 6: Inhibition rates of Example 4 on different hTRPs (0.3 µ M)**

| Ion channel name | Example 4 | | Test method |
|---|---|---|---|
| | Inhibition rate (%) | Selectivity ratio | |
| hTRPV3 | 72.80 | 1x | Patch clamp method |
| hTRPV1 | 0.48 | 152x | Patch clamp method |
| hTRPV4 | 36.21 | 2x | Patch clamp method |
| hTRPA1 | 2.44 | 30x | Patch clamp method |

| | | | |
|---|---|---|---|
| Note: The selectivity ratio relates to the ratio of the inhibition rate of a compound on TRPV3 ion channels to the inhibition rate on other ion channels. | | | |

### Liver microsome metabolism stability test

Two separate experiments were conducted. a) NADPH: 10 µL of 20 mg/mL liver microsomes and 40 µL of 10mM NADPH were added to a culture medium. The final concentrations of microsomes and NADPH were 0.5 mg/mL and 1 mM, respectively. b) NADPH free: 10 µL of 20 mg/mL liver microsomes and 40 µL of ultrapure H₂O were added to a culture medium. The final concentration of microsomes was 0.5 mg/mL.

At the beginning of the reaction, 4 µL of 100 µM test compound solution or a control compound solution with a final concentration of 1 µM were added, and proceed at 37 °C.

50 µL aliquots were taken from the reaction solution at 0, 7, 15, 30, and 60 minutes. The reaction was stopped by adding 4 volumes of cold acetonitrile and IS (100 nM alprazolam, 200 nM labetalol, 200 nM caffeine, and 2 µM ketoprofen). The samples were centrifuged at 3220 grams for 40 minutes. 100 µL supernatant divided equally was mixed with 100 µL of ultrapure H₂O, and use for LC-MS/MS analysis.

**Table 7: Metabolic stability test results of liver microsomes**

| Compound No. | Genus | *in vitro* T_{1/2} (min) | *in vitro* Clᵢₙₜ (microliters/minute/milligrams of protein) |
|---|---|---|---|
| Example 1 | human | 10.27 | 135.00 |
| | rat | 12.36 | 112.14 |

Although preferred embodiments of the present invention have been disclosed to illustrate the present invention, those skilled in the art should understand that various modifications, additions, and substitutions can be made to the present invention without departing from the inventive concept and scope as defined in claims.

## Claims

1. A compound of formula (I), or stereoisomers, tautomers, solvates, hydrates, active metabolites, isotopic labels, and pharmaceutically acceptable salts thereof: 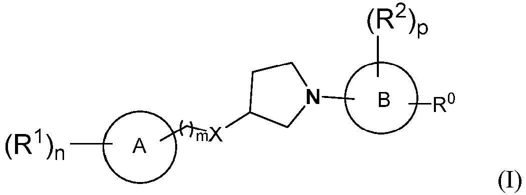
wherein ring A and ring B are each independently selected from a monocyclic or polycyclic ring system containing 3-12 ring atoms;
R¹ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}), -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R², together with the atoms of ring B to which they are connected, form a 3-10 membered ring structure;
R⁰ is each independently selected from the group consisting of H, halogen or a structure of formula II 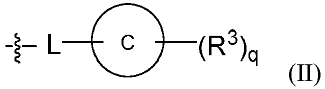
wherein,
L is each independently selected from the group consisting of a bond, -O-, -S-, -N(R²⁰)-, - C(O)-, -C(R²⁰R²¹)-, -S(O)- and -S(O₂)-;
ring C is each independently selected from a monocyclic or polycyclic ring system containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f};
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f};
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
X is selected from the group consisting of -S-, -S(O)-, and -S(O₂)-;
n is 0, 1, 2 or 3;
m is 0, 1, 2 or 3;
p is 0, 1, 2 or 3; and
q is 0, 1, 2 or 3.

2. The compound according to claim 1, wherein the compound has a structure of formula I-1 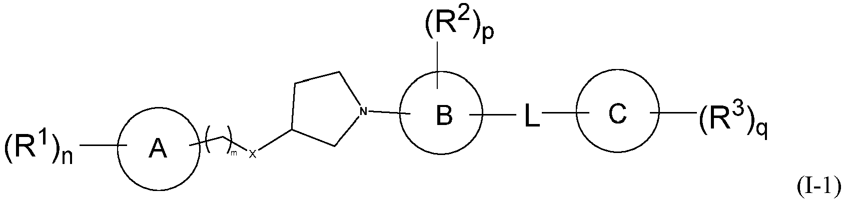 wherein ring A, ring B, ring C, X, R¹, R², R³, L, n, p, m, and q are defined as formula I.

3. The compound according to claim 1 or 2, wherein ring A is selected from the group consisting of benzene ring, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

4. The compound according to claim 3, wherein ring A is selected from the following structural formulae:

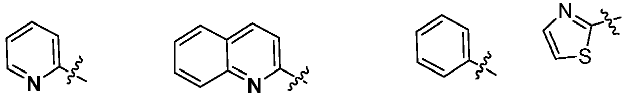

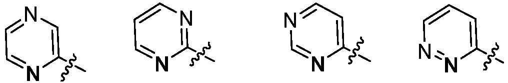

5. The compound according to claim 1 or 2, wherein ring B is selected from the group consisting of benzene ring, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

6. The compound according to claim 5, wherein ring B is selected from the following structural formulae:

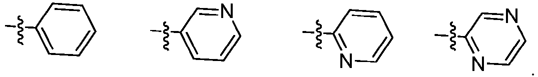

7. The compound according to claim 1 or 2, wherein R¹ is each independently selected from the group consisting of H, halogen, cyano, hydroxyl, C₁-C₆ alkyl, C₁-C₆ haloalkyl, C₁-C₆ alkoxy, -N(R^{a})(R^{b}) and -R¹¹OR¹², wherein R^{a} and R^{b} are each independently selected from the group consisting of H and C₁-C₆ alkyl; R¹¹ is each independently selected from C₁-C₆ alkylene; R¹² is each independently selected from the group consisting of H, and C₁-C₆ alkyl.

8. The compound according to claim 7, wherein R¹ is each independently selected from the group consisting of H, Cl, F, -CF₃, -CN, -CH₃, -OH, -OCH₃, and -CH₂OCH₃.

9. The compound according to claim 1 or 2, wherein R² is each independently selected from the group consisting of H, cyano, hydroxyl, C₁-C₆ alkyl, C₁-C₆ haloalkyl, C₃-C₆ cycloalkyl substituted with 0 to 2 R^{f}, C₁-C₆ alkoxy, C₁-C₆ haloalkoxy, -R¹¹OR¹², -R¹¹SR¹², -C(O)OR^{d} and -C(O)N(R^{a})(R^{b}); wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{d} is each independently selected from the group consisting of H and C₁-C₆ alkyl; R^{f} is each independently selected from the group consisting of halogen, hydroxyl, amino and C₁-C₆ alkyl.

10. The compound according to claim 9, wherein R² is each independently selected from the group consisting of H, -CH₃, -OH, -CH₂OH, -CH₂OCH₃, -CH₂CH₂OH, -CH(CH₃)OH, - C(CH₃)₂OH, -CH₂SH, -OCH₂CH₃, -OCF₃, -OCH₃, -OCH(CH₃)₂, -COOH, -COOCH₃, -CONH₂, -CH₂NH₂, -CH₂CHF₂, -CN, -CHF₂, 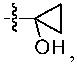 and

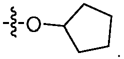

11. The compound according to claim 2, wherein L is each independently selected from the group consisting of a bond, -O-, -S-, -N-, -C(O)-, -CH₂-, -C(OH)-, -S(O)-, and -S(O₂)-.

12. The compound according to claim 2, wherein ring C is selected from the group consisting of C₃-C₆ cycloalkane ring, benzene ring, benzo-C₃-C₆ cycloalkane rings, C₅-C₁₂ bridged carbocyclic ring, pyridine ring, quinoline ring, isoquinoline ring, pyrazine ring, pyrimidine ring, pyridazine ring, thiazole ring, thiophene ring, pyrrole ring, pyrazole ring, imidazole ring, isothiazole ring, indole ring, benzimidazole ring, furan ring, oxazole ring, quinoxaline ring, and purine ring.

13. The compound according to claim 12, wherein ring C is selected from the following structural formulae:

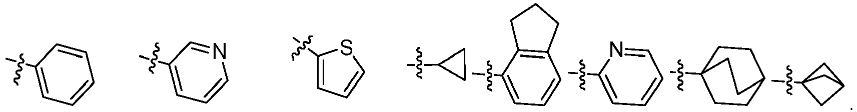

14. The compound according to claim 2, wherein R³ is each independently selected from the group consisting of H, cyano, hydroxyl, halogen, C₁-C₆ alkyl, C₁-C₆ haloalkyl, C₁-C₆ alkoxy, C₁-C₆ haloalkoxy, C₃-C₆ cycloalkyl, -R¹¹OR¹², -R¹¹SR¹², -C(O)R^{c}, -C(O)OR^{d}, and - C(O)N(R^{a})(R^{b}), or two R³, together with the atoms of ring C to which they are connected, form a 3-10 membered ring structure;
wherein R¹¹ is each independently selected from C₁-C₆ alkylene; R¹² is each independently selected from the group consisting of H, C₁-C₆ alkyl, C₁-C₆ haloalkyl, C₃-C₆ cycloalkyl, and C₃-C₆ halocycloalkyl; R^{a} and R^{b} are each independently selected from the group consisting of H and C₁-C₆ alkyl; R^{c} is each independently selected from the group consisting of H, halogen, and C₁-C₆ alkyl; R^{d} is each independently selected from the group consisting of H and C₁-C₆ alkyl.

15. The compound according to claim 14, wherein R³ is each independently selected from the group consisting of H, cyclopropyl, isopropyl, tert-butyl, F, Cl, ethyl, methyl, methylcarbonyl, and methoxymethyl.

16. The compound according to claim 1 or 2, wherein m is 0.

17. The compound according to claim 1 or 2, wherein n is 0, 1 or 2.

18. The compound according to claim 1 or 2, wherein n is 1 and m is 0, and R¹ is located at the ortho or para position of X.

19. The compound according to claim 1 or 2, wherein n is 2 and m is 0, and one R¹ is located at the para position of X.

20. The compound according to claim 1 or 2, wherein p is 1, preferably the R² is located at the ortho position of pyrrolidinyl.

21. The compound according to claim 1 or 2, wherein R² is -CH₂OH.

22. The compound according to claim 1 or 2, wherein L is -S-; or
L is -S(O)-; or
L is -S(O₂)-;
preferably, L is -S-.

23. A compound of formula (I-2), or stereoisomers, tautomers, solvates, hydrates, active metabolites, isotopic labels, and pharmaceutically acceptable salts thereof: 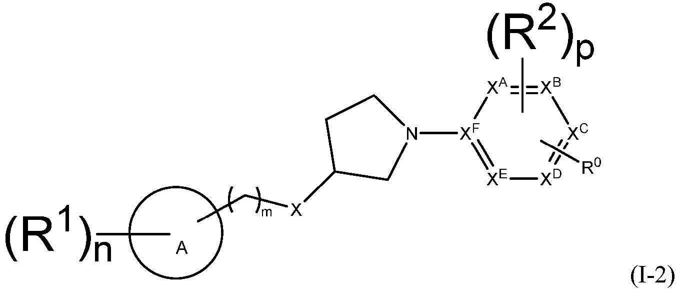
wherein ring A is each independently selected from a monocyclic or polycyclic ring system containing 3-12 ring atoms;
R¹ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, - C(O)N(R^{a})(R^{b}) and -SO₂N(R^{a})(R^{b}), and -SOR^{c}, or two R¹, together with the atoms of ring A to which they are connected, form a 3-10 membered ring structure;
R² is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R², together with the atoms of the ring to which they are connected, form a 3-10 membered ring structure;
R⁰ is each independently selected from the group consisting of H, halogen or a structure of formula II 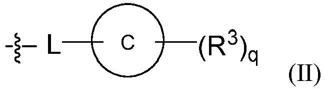
wherein L is each independently selected from the group consisting of a bond, -O-, -S-, - N(R²⁰)-, -C(O)-, -C(R²⁰R²¹)-, -S(O)- and -S(O₂)-;
ring C is each independently selected from a monocyclic or polycyclic ring system containing 3-12 ring atoms;
R³ is each independently selected from the group consisting of H, halogen, hydroxyl, mercapto, nitro, cyano, oxo, C₁-C₆ alkyl substituted with 0 to 2 R^{f}, C₁-C₆ haloalkyl substituted with 0 to 2 R^{f}, C₁-C₆ alkoxy substituted with 0 to 2 R^{f}, C₁-C₆ haloalkoxy substituted with 0 to 2 R^{f}, C₃-C₆ cycloalkyl substituted with 0 to 2 R^{f}, C₃-C₆ halocycloalkyl substituted with 0 to 2 R^{f}, C₃-C₆ cycloalkoxy substituted with 0 to 2 R^{f}, C₃-C₆ halocycloalkoxy substituted with 0 to 2 R^{f}, aryl substituted with 0 to 2 R^{f}, aralkyl substituted with 0 to 2 R^{f}, alkaryl substituted with 0 to 2 R^{f}, heteroaryl substituted with 0 to 2 R^{f}, -R¹¹OR¹², -R¹¹SR¹², -N(R^{a})(R^{b}), -C(O)R^{c}, -C(O)OR^{d}, - C(O)N(R^{a})(R^{b}), -SO₂N(R^{a})(R^{b}), and -SOR^{c}, or two R³, together with the atoms of ring C to which they are connected, form a 3-10 membered ring structure;
R¹¹ is each independently selected from C₁-C₆ alkylene substituted with 0 to 2 R^{f};
R¹² is each independently selected from the group consisting of H, C₁-C₆ alkyl substituted with 0 to 2 R^{f}, C₁-C₆ haloalkyl substituted with 0 to 2 R^{f}, C₃-C₆ cycloalkyl substituted with 0 to 2 R^{f}, and C₃-C₆ halocycloalkyl substituted with 0 to 2 R^{f};
R^{a} and R^{b} are each independently selected from the group consisting of H, C₁-C₆ alkyl substituted with 0 to 2 R^{f}, aryl substituted with 0 to 2 R^{f}, aralkyl substituted with 0 to 2 R^{f}, - C(O)R^{c}, and -C(O)OR^{d};
R^{c} is each independently selected from the group consisting of H, halogen, C₁-C₆ alkyl substituted with 0 to 2 R^{f}, aryl substituted with 0 to 2 R^{f}, and aralkyl substituted with 0 to 2 R^{f};
R^{d} is each independently selected from the group consisting of H, C₁-C₆ alkyl substituted with 0 to 2 R^{f}, aryl substituted with 0 to 2 R^{f}, and aralkyl substituted with 0 to 2 R^{f};
R²⁰ and R²¹ are each independently selected from the group consisting of H, hydroxyl, C₁-C₆ alkyl, aryl, and aralkyl;
R^{f} is each independently selected from the group consisting of halogen, hydroxyl, amino, C₁-C₆ alkyl, C₁-C₆ alkoxy, C₁-C₆ haloalkyl, C₁-C₆ haloalkoxy, C₃-C₆ cycloalkyl, and C₃-C₆ halocycloalkyl;
X is selected from the group consisting of -S-, -S(O)-, and -S(O₂)-;
X^{A}, X^{B}, X^{C} X^{D} and X^{E} are each independently CH or N;
X^{F} is CH;
n is 0, 1, 2 or 3;
m is 0, 1, 2 or 3;
p is 0, 1, 2 or 3;
q is 0, 1, 2 or 3.

24. The compound according to claim 23, wherein
X^{A}, X^{B}, X^{C}, X^{D}, X^{E} are each independently CH; or
X^{A}, X^{B}, X^{C} X^{E} are each independently CH, and X^{D} is N; or
X^{A}, X^{B}, X^{C} X^{D} are each independently CH, and X^{E} is N; or
X^{A}, X^{C}, X^{D} are each independently CH, and X^{B} and X^{E} are N.

25. The compound according to claim 1, wherein the compound is selected from the following compounds:

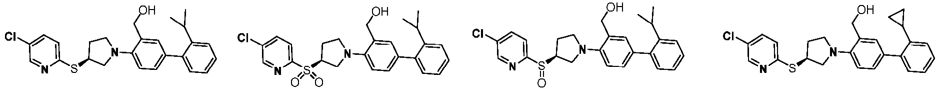

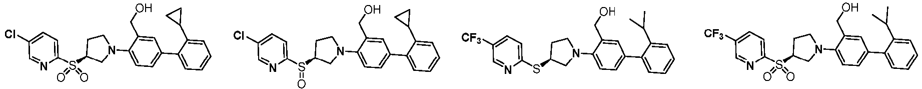

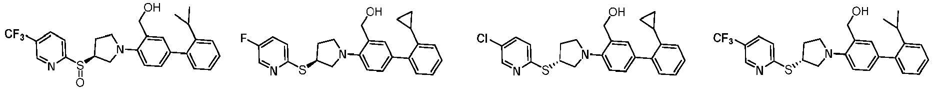

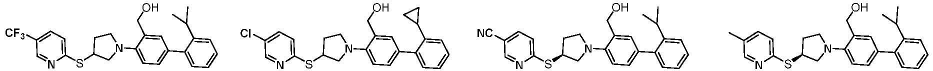

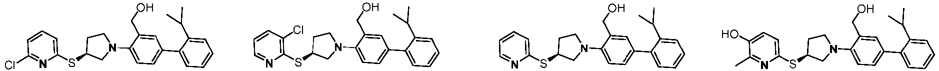

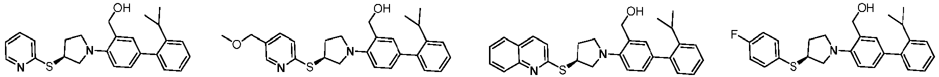

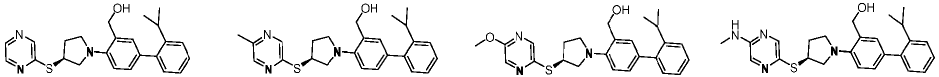

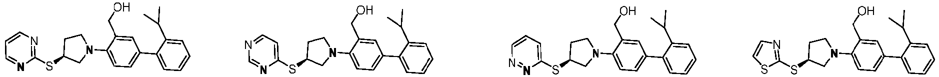

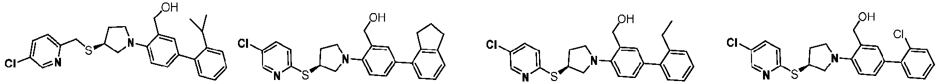

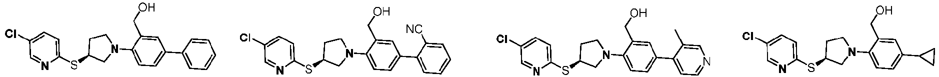

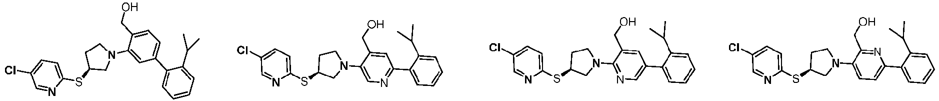

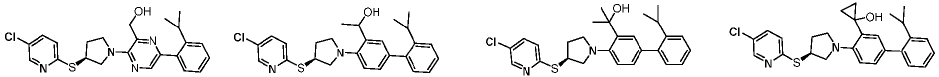

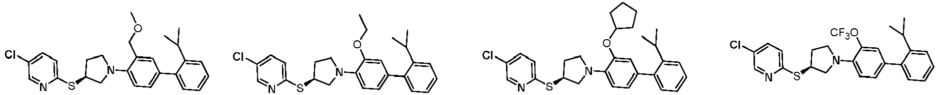

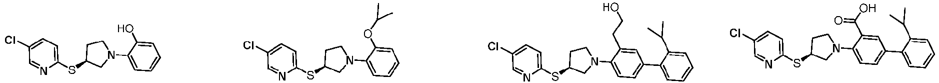

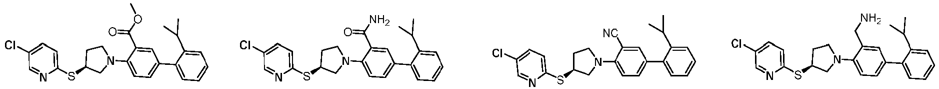

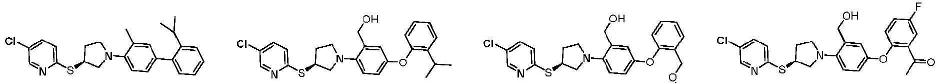

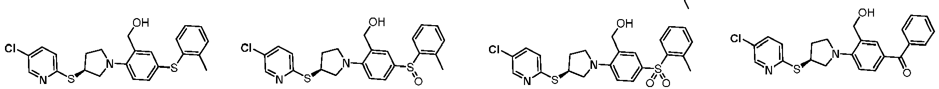

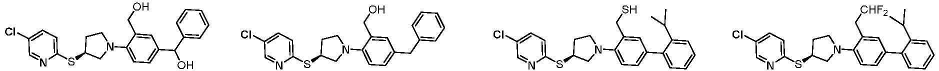

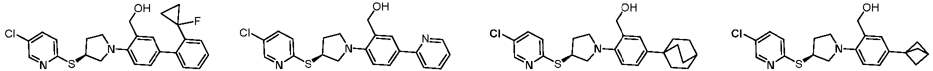

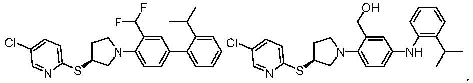

26. A pharmaceutical composition, comprising the compound according to any one of claims 1 to 25, or stereoisomers, tautomers, solvates, hydrates, active metabolites, isotopic labels, or pharmaceutically acceptable salts thereof, and a pharmaceutically acceptable carrier.

27. Use of the compound according to any one of claims 1 to 25, or stereoisomers, tautomers, solvates, hydrates, active metabolites, isotopic labels, or pharmaceutically acceptable salts thereof, and the pharmaceutical composition according to claim 26 in manufacture of pharmaceuticals for inhibiting TRPV3 activity.

28. Use of the compound according to any one of claims 1 to 25, or stereoisomers, tautomers, solvates, hydrates, active metabolites, isotopic labels, or pharmaceutically acceptable salts thereof, and the pharmaceutical composition according to claim 26 in manufacture of pharmaceuticals for treating TRPV3-mediated conditions in a subject.

29. Use according to claim 27, wherein the conditions are selected from the group consisting of pain, itching, skin conditions, inflammation, abnormal hair growth, incontinence, fever, hot flashes, cystitis, irritable bowel syndrome, and/or cough symptoms.

30. Use according to claim 29, wherein the pain is cancerous pain and skin pain.

31. Use according to claim 30, wherein used for manufacturing pharmaceuticals that inhibit proliferation, and thereby prevent, treat, or alleviat cancer symptoms.

32. Use according to claim 31, wherein the cancer is liposarcoma.

33. Use according to claim 29, wherein the abnormal hair growth is hair loss.

34. Use according to claim 29, wherein the skin conditions are selected from the group consisting of skin keratosis, ichthyosis, and pruritus.

35. Use according to claim 34, wherein the skin keratosis is olmsted syndrome.

36. Use according to claim 35, wherein the ichthyosis is harlequin ichtyosis.

37. A method for treating TRPV3 mediated conditions, comprsing administering a therapeutically effective amount of the compound according to any one of claims 1 to 25, or stereoisomers, tautomers, solvates, hydrates, active metabolites, isotopic labels, or pharmaceutically acceptable salts thereof, or the pharmaceutical composition according to claim 26 to a patient in need of administration.
